# EUROPEAN PATENT APPLICATION

(11) **EP 4 732 907 A1**
(43) Date of publication of application: **29.04.2026**
(21) Application number: 24383173.2
(22) Date of filing: 25.10.2024
(51) Int. Cl.: A61P 33/02, C07D 277/62, C07D 471/06, A61K 31/428

(54) **STYRYL BENZOTHIAZOLIUM SALTS AND ITS USES AGAINST PARASITIC INFECTIONS**

(71) Applicant: Consejo Superior de Investigaciones Científicas (CSIC), 28006 Madrid (ES)
(72) Inventor: Morales Sánchez, Juan Carlos, Granada (ES); Peñalver Puente, Pablo, Granada (ES); Pérez-Victoria Moreno de Barreda, José María, Granada (ES); Raposo Marques, Maria Manuela, Braga (PT); Rainha da Costa Gonçalves, Raquel, Braga (PT); Graça Costa, Susana Paula, Braga (PT); García Pérez, Belén, Granada (ES)
(74) Representative: Pons IP

(57) **Abstract**

The present invention refers to benzothiazolium salts of formula (I) and its use in therapy. More particularly, they are useful in the treatment and/or prevention of parasitic infections. The present invention also relates to pharmaceutical composition comprising them.

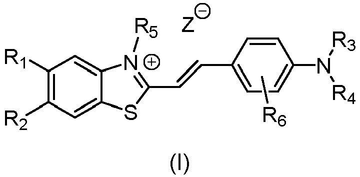

## Description

The invention relates to novel compounds, which are styryl benzothiazolium salts, and its use in therapy; particularly they have shown antitrypanosomal and leishmanicidal activity.

Therefore, the present invention belongs to the field of medicine and veterinary science.

### BACKGROUND ART

Trypanosomiasis and Leishmaniasis are considered neglected tropical diseases that affect humans and other animals. They are caused by infections of *Trypanosoma brucei, Trypanosoma cruzi* and *Leishmania spp.* Specifically, these parasites cause human African trypanosomiasis or sleeping sickness, American trypanosomiasis or Chagas disease and leishmaniasis (in its different clinical manifestations: visceral, cutaneous and mucocutaneous), respectively. These protozoa have life cycles that alternate between two different hosts, an insect vector and the mammalian host. In the insect, the procyclic (*T. brucei*), epimastigote (*T. cruzi*) and promastigote (*Leishmania*) forms are extracellular, whereas, in the mammalian host, the amastigotes forms of Leishmania spp. and *T. cruzi* progress intracellularly, except for the *T. brucei* parasites which are extracellular. The actual treatments against these diseases consist of drugs that are toxic, outdated and non-efficient for patients suffering these diseases.

The benzothiazole core has been recognized as an important scaffold with a wide range of pharmacological properties including anticancer, anti-inflammatory, antidiabetic, anticonvulsant, antioxidant, antiviral, and antimicrobial activity (Yadav et al., Intelligent Pharmacy 2023, 1, 122-132).

In document Velásquez-Torres et al., Pharmaceuticals 2023, 16, 896, a derivative of benzothiazole as a potential anti-amoebic agent against *Entamoeba Histolytica* is reported.

In document Cuevas-Hernández et al., Antimicrob Agents Chemother 2020, 64, e01742-19, a fluorinated phenylbenzothiazole arrests the *Trypanosoma Cruzi* cell cycle and diminishes the Infection of mammalian host cells is reported.

In document Martínez-Cerón, et al., Parasitol Res 2021, 120, 2905-2918, phenylbenzothiazole derivatives show effects against a *Trypanosoma Cruzi* Infection.

In document, Dunn, et al., J. Med. Chem. 1966, 9, 751-753, 2-(nitro-heterocyclic) benzothiazoles were shown to be potent inhibitors of *Trichomonas foetus* in vitro, and several compounds also had anthelmintic activity in a natural mouse pinworm infection.

In document, Haugwitz, et al., J. Med. Chem. 1982, 25, 969-974, 2-heteroaromatic-substituted isothiocyanatobenzoxazoles and -benzothiazoles were reported to show anthelmintic activities.

In document, Lapierre et al., Chem Biol Drug Des. 2024; 103: e14525, 2-amido-benzothiazole derivatives showed relevant efficiency against *Trypanosoma brucei* and *Leishmania infantum* parasites together with low cytotoxicity against THP-1 macrophages.

In document, Chabrecek et al., Chemical Papers (1987), 41(5), 655-69, 2-styrylbenzothiazolium salts substituted at position 3 with antibacterial and antifungal activity were reported.

In document, CN109912532, 2-styrylbenzothiazolium salts and its application in preparing bacterial biomembrane inhibitors were reported.

In document, Eibergen, et al., ChemBioChem (2012), 13(4), 574-583, the identification of a benzothiazolium salt, ABTZ-1, and derivatives that displayed potent antibacterial activity against Gram-positive pathogens was reported.

In document, Sigmundova et al., ARKIVOC (2008), (8), 183-192, new 2-styryl benzothiazolium salts substituted on the heterocyclic ring with antimicrobial activity were described.

In document, Radovan et al., Collection of Czechoslovak Chemical Communications (2002), 67(12), 1820-1832, a series of new push-pull 2-(w-phenylalkenyl) benzothiazolium compounds with methyl groups in different positions of the conjugated alkenyl bridge and antimicrobial activity were reported.

In document, Magdolen, et al., Conference of Organic Chemists on Advances in Organic Chemistry, 22nd, Casta-Papiernicka, Slovakia, June 11-13, 1997, 185, a family of 2-styrylbenzothiazolium salts with activity against *Mycobacterium tuberculosis* was described.

In document, Kovalenko, F. P. **1978,** 15 pp, a family of 39 2-styrylbenzothiazolium salts with anthelmintic activity against *Alveococcus multilocularis* and *Echinococcus granulosus* was reported.

In document, Kortisova, et al., J. Chemical Papers (2006), 60(1), 52-55, compounds based on 2-ethynylbenzothiazolium salts with a furane unit exhibited significant activities against several bacterial, yeast, and mould strains. The effect on growth and on the plastid system of the unicellular flagellate *Euglena gracilis* was also studied.

In document Jian-Feng Ge, et al. Med. Chem. Commun., 2012, 3, 1435-1442, the synthesis of cyanine dyes and investigation of their in vitro antiprotozoal activities was described, specifically against *Plasmodium falciparum K1, Trypanosoma cruzi, T. brucei rhodesiense and Leishmania donovani.* Among them, trimethine cyanines containing benzothiazolyl groups exhibited strong activities against *P. falciparum* and *T. cruzi,* whilst one symmetric pentamethine cyanine containing a fluorinated benzothiazolyl group was highly active against *T. b. rhodesiense.*

Currently there is no effective prophylactic method to prevent diseases caused by these parasites and chemotherapeutic treatments do not have the desired effectiveness, are toxic and/or are threatened by the appearance of therapeutic failure and resistance. New drugs are therefore urgently needed.

Despite continued attempts to control and treat leishmaniasis, the emergence of drug-resistant strains presents a serious problem that weakens the effectiveness of current treatment strategies. The drugs available to treat leishmaniasis are mainly pentavalent antimonials, the first line of treatment despite their toxicity and low effectiveness due to the easy appearance of resistance, amphotericin B, which requires parenteral administration, is too expensive in its liposomal form, the only one that is not very toxic, and is threatened by resistance and miltefosine, the only oral drug to treat the disease but which is not effective against all species of leishmania, is teratogenic, too expensive and cases of resulting resistance have already appeared. Of its long half-life (7 days) which causes the existence of subtherapeutic doses over time.

In the case of patients suffering Chagas only two drugs are approved, the first-line drug Benznidazole and the second-line drug Nifurtimox. They are effective in the acute phase of the disease and in congenital transmission. The administration is also recommended in the chronic phase, although the cure rate importantly decreases. At the same time, both of them cause serious side effects due to their high toxicity what leads to frequent treatment abandonment by the patients.

In view of the prior art, there is a need to provide improved antiparasitic agents. In particular, there is a need to provide further benzothiazole derivatives, which have improved antitrypanosomal effects.

### SUMMARY OF THE INVENTION

The present invention discloses novel compounds, which are benzothiazolium salts modified with a styryl group at position 2 that incorporate an amino group at position 4 of the phenyl ring. The invention also concerns pharmaceutical compositions comprising the novel compounds and their use in therapy.

A first aspect of the present invention relates to a compound of general formula (I), or a pharmaceutically acceptable salt thereof: 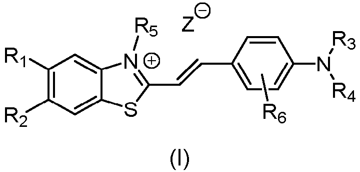 wherein
R₁ and R₂ are each independently selected from H, halogen, -O-(C₁-C₄)-alkyl or C₁-C₄ alkyl,
R₃ and R₄ are each independently selected from:
   optionally substituted C₁-C₈ alkyl and phenyl,
   R₃ and R₄ form a five- or six-membered ring with the N to which they are bonded and with the phenyl ring that is bonded to that N, and
   R₃ and R₄ form, together with the N to which they are bonded, a five- or six-membered aliphatic or aromatic ring,
R₅ is an optionally substituted C₁₋₈ alkyl,
R₆ is a H, halogen or a optionally substituted C₁₋₈ alkyl,
Z is a counterion to offset the positive charge of the nitrogen of the aromatic ring being zero the final charge of the compound of formula (I),
for use in the treatment and/or prevention of parasitic infections.

In one embodiment, R₁ and R₂ are each independently H or halogen, more preferably the halogen is chlorine or fluorine, and even more preferably the halogen is fluorine.

In another embodiment, R₃ and R₄ are each independently C₁-C₈ alkyl, more preferably C₁-C₄ alkyl, and even more preferably ethyl or methyl.

In a preferred embodiment, R₃ and R₄ are each independently optionally substituted phenyl (Ph). If substituted, the substituents of the Ph groups are selected preferably from: C₁-C₄ alkyl, methoxy, halogen, amino (-NH₂), nitro (-NO₂) or combinations thereof. Preferably, the Ph group is unsubstituted.

In another embodiment, R₅ is independently C₁-C₈ alkyl, more preferably C₁-C₄ alkyl, and even more preferably propyl, ethyl or methyl.

In another embodiment, R₆ is independently H, halogen or C₁-C₈ alkyl and more preferably is a H or F.

In another embodiment, R₆ is in position *orto* with respect to the -NR₃R₄ group.

In a preferred embodiment, Z⁻ is selected from: halide, acetate, besilate, benzoate, carbonate, bicarbonate, benzenesulphonate, bitartrate, citrate, estearate, phosphate, fumarate, glucuronate, glycolate, hexanoate, hydroxynaphtoate, lactate, mesylate, nitrate, nitrite, octanoate, oleate, pamoate, pantotenate, propionate, poligalacturonate, salicilate, succinate, sulphate, tartrate, teoclate, tosylate, triflate, tetraphenylborate y trifluoroacetate. More preferably, Z⁻ is a halide (F⁻, Cl⁻, Br⁻ or I⁻), even more preferably, Z⁻ is I⁻.

In another embodiment, the preferred compounds of formula (I) for use are selected from the group:

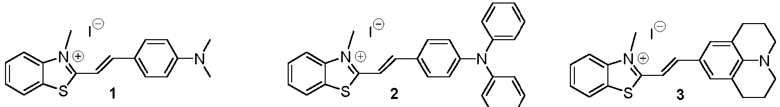

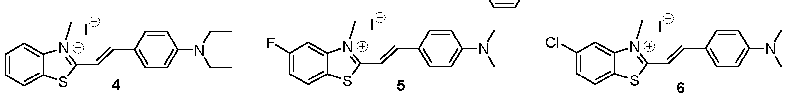

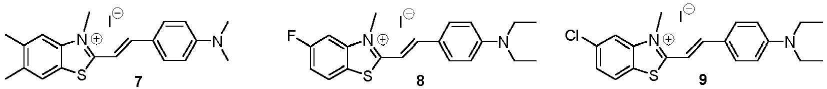

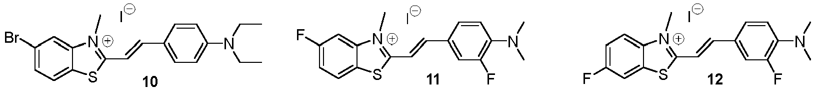

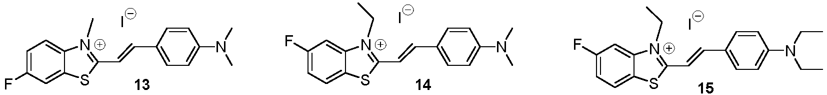

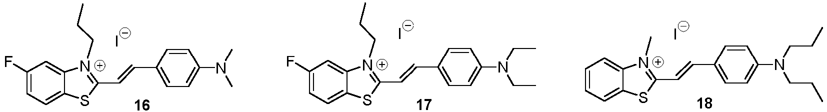

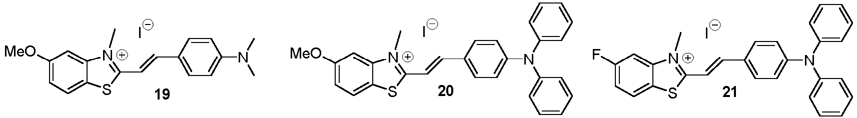

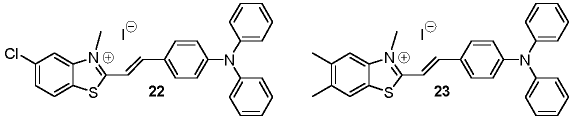

In a preferred embodiment, the parasitic infection is selected from: african trypanosomiasis, chagas, leishmaniasis, blastocystosis, cryptosporidiosis, amoebiasis, giardiasis, sarcosystosis, toxoplasmosis, trichomoniasis and malaria. More preferably, the parasitic infection is selected from african trypanosomiasis, chagas, leishmaniasis and malaria.

In another preferred embodiment, the infection is leishmaniasis caused by *Leishmania major, Leishmania donovani* or *Leishmania infantum* parasites.

A second aspect of the invention refers to a compound of formula (I) described above, wherein the compound is selected from the next ones:

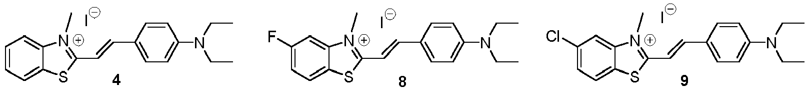

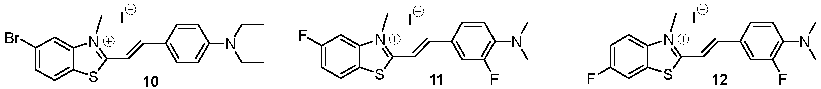

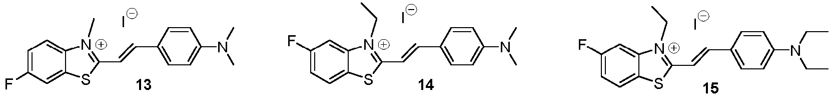

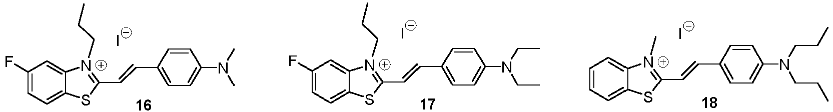

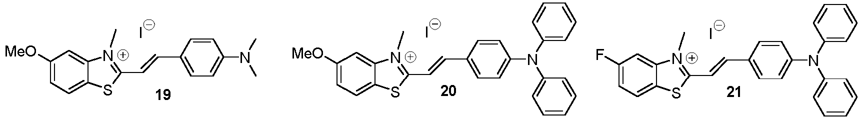

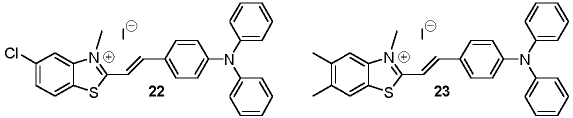

Another aspect of the invention relates to the compound of formula (I), or to a pharmaceutically acceptable salt thereof, as defined in the second aspect of the invention, for use as a medicament.

In another aspect, the present invention relates to a pharmaceutical composition comprising at least one compound of formula (I) or a pharmaceutically acceptable salt thereof, as defined in the second aspect of the invention, together with pharmaceutically acceptable excipients, adjuvants and/or carriers, for the administration to a patient.

The pharmaceutically acceptable adjuvants and vehicles that may be used in said compositions are the adjuvants and vehicles known to those skilled in the art and are usually used to prepare therapeutic compositions.

The compounds described in the present invention, the salts thereof, as well as the pharmaceutical compositions containing them can be used together with other additional drugs, or active ingredients, in order to provide a combination therapy. Said additional drugs can make up part of the same pharmaceutical composition or, alternatively, they can be provided in the form of a separate composition for the administration thereof which is simultaneous or not to that of the pharmaceutical composition comprising a compound of formula (I), or a salt thereof.

In a particular embodiment, the pharmaceutical composition is prepared in a solid form or aqueous suspension, in a pharmaceutically acceptable diluent. The therapeutic composition provided by this invention can be administered by any suitable route of administration, for which said composition will be formulated in the pharmaceutical form suitable for the chosen route of administration. In a particular embodiment, the administration of the therapeutic composition provided by this invention is performed by the following routes: oral, topical, rectal or parenteral (including subcutaneous, intraperitoneal, intradermal, intramuscular, intravenous, etc.).

In a preferred embodiment of the present invention, the pharmaceutical compositions are suitable for oral administration, in solid or liquid form. The possible forms for oral administration are tablets, capsules, syrups or solutions and may contain conventional excipients known in the pharmaceutical field, such as aggregating agents (e.g., syrup, acacia, gelatine, sorbitol, tragacanth or polyvinylpyrrolidone), fillers (e.g., lactose, sugar, corn starch, calcium phosphate, sorbitol or glycine), disintegrants (e.g., starch, polyvinylpyrrolidone or microcrystalline cellulose) or a pharmaceutically acceptable surfactant such as sodium lauryl sulphate.

The compositions for oral administration can be prepared by conventional galenic pharmacy methods, such as mixing and dispersing. The tablets can be coated following methods known in the pharmaceutical industry.

The pharmaceutical compositions can be adapted for parenteral administration, as sterile solutions, suspensions, or lyophilised solutions of the products of the invention, using the suitable dose. Suitable excipients can be used, such as pH buffering agents or surfactants.

The aforementioned formulations can be prepared using conventional methods, such as those described in the Pharmacopoeias of different countries and in other reference texts.

The administered amount of a compound of the present invention will depend on the relative efficacy of the chosen compound, the severity of the disease to be treated and the weight of the subject. The term "subject" in the present invention includes both humans and non-human animals.

All the compounds described in the invention can be in crystalline form as free compounds or as solvates. In this sense, the term "solvate", as used here, includes both pharmaceutically acceptable solvates, in other words, solvates of the compound of formula (I) which can be used in the preparation of a medicine, and pharmaceutically unacceptable solvates, which can be used in the preparation of pharmaceutically acceptable salts or solvates. The nature of the pharmaceutically acceptable solvate is not critical as long as it is pharmaceutically acceptable. In a particular embodiment, the solvate is a hydrate. The solvates can be obtained by conventional solvation methods known to those skilled in the art.

For the application thereof in therapy, the compounds of formula (I) of the present invention, the salts or solvates thereof, will be found, preferably, in a pharmaceutically acceptable or substantially pure form, in other words, having a pharmaceutically acceptable purity level excluding normal pharmaceutical additives such as diluents and carriers, and not including material considered toxic at normal dosage levels. The purity levels for the active ingredient are preferably greater than 50%, more preferably greater than 70%, and still more preferably greater than 90%. In a preferred embodiment, they are greater than 95% of the compound of formula (I) or the salts or solvates thereof.

Another aspect of the invention relates to a method of treating and/or preventing parasitic infections, said method comprising administering to a subject a therapeutically effective amount of a compound of formula (I) or a pharmaceutically acceptable salt thereof, wherein R₁ to R₆ are as defined in the first aspect of the invention.

A last aspect of the present invention relates to the use of a compound of formula (I) or to a pharmaceutically acceptable salt thereof, wherein R₁ to R₆ are as defined in the first aspect of the invention for the preparation of a medicament for use in the treatment and/or prevention of parasitic infections.

### Definitions

The term "alkyl", in the present invention, refers to a saturated, linear or branched hydrocarbon chain, which bind to the rest of the molecule by a single bond. The number of carbons of the chain is indicated next to the word alkyl. Thus, for example, C₁-C₄ alkyl refers to a saturated, linear or branched hydrocarbon chain having between 1 to 4 carbon atoms. Examples of "alkyl" are: methyl, ethyl, *n*-propyl, *i*-propyl, *n*-butyl, *tert*-butyl, *sec-*butyl, etc. Alkyl groups may be optionally substituted by one or more substituents. Non limiting substituents are, for example, halogens, ciano and methoxy.

Preferably, the alkyl groups present in formula (I) are unsubstituted.

The term "halogen" refers to F, Cl, Br or I.

The term "excipients, adjuvants and/or carriers" relates to molecular entities or substances through which the active ingredient is administered. Such pharmaceutical excipients, adjuvants or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are known for the skilled person in the art.

The term "pharmaceutically acceptable salts" relates to salts or solvates which, on being administered to the recipient, are capable of providing a compound such as that described herein. The preparation of salts and derivatives can be carried out by methods known in the state of the art. Preferably, "pharmaceutically acceptable" relates to molecular entities and compositions that are physiologically tolerable and do not typically produce an allergic reaction or a similar unfavourable reaction, such as gastric upset, dizziness and similar side effects, when administered to a human.

In the sense used in this description, the expression "therapeutically effective amount" refers to the amount of the agent or compound capable of developing the therapeutic action determined by the pharmacological properties thereof, calculated to produce the desired effect and, in general, it will be determined, among other causes, by the very features of the compounds, including the age, state of the subject, the severity of the illness or disorder, and the route and frequency of administration.

The term "treatment or prevention" as used herein, unless otherwise indicated, relates to reversing, alleviating and inhibiting the progress of, or preventing the disorder or condition to which it applies in such terms, one or more symptoms of such disorder or condition.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to be limiting of the present invention.

### EXAMPLES

### Example 1. Synthesis of styryl benzothiazolium salts

For the synthesis of the benzothiazolium precursors: To a solution of commercial 2-methyl benzothiazole (3 mmol) in acetonitrile (5 mL) was added methyl, ethyl or *n-*propyl iodide (18 mmol). The solution was stirred and heated at reflux (80 °C) for 6-96 h. The precipitate was filtered and washed with cold acetonitrile to give the pure product.

As an example, the synthesis of 2,3-dimethylbenzo[d]thiazol-3-ium iodide

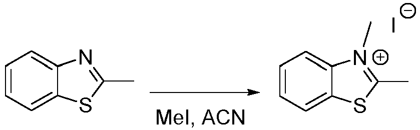

**Table 1. Commercial benzothiazoles and alkyl iodides for the synthesis of the benzothiazolium precursors.**

| **Commercial benzothiazole** | **Alkyl iodide** | **Benzothiazolium precursor** | **Ref.** |
|---|---|---|---|
| 2-methylbenzo[*d*]thiazole | methyl iodide | 2,3-dimethylbenzo[*d*]thiazol-3-ium iodide | Coelho, et al., Dyes and Pigments (2015), 117, 163-169 |
| 5-fluoro-2-methylbenzo[*d*]thiazole | methyl iodide | 5-fluoro-2,3-dimethylbenzo[*d*]thiazol-3-ium iodide | Renard, et al., Tetrahedron Letters (2009), 50 (17), 1897-1901 |
| 5- chloro-2-methylbenzo[*d*]thiazole | methyl iodide | 5-chloro-2,3-dimethylbenzo[*d*]thiazol-3-ium iodide | Sun, et al., Dyes and Pigments (2013), 96(1), 189-195 |
| 2,5,6-trimethylbenzo[*d*]thiazole | methyl iodide | 2,3,5,6-tetramethylbenzo[*d*]thiazol-3-ium iodide | Mallesh, et al., ACS Chem. Neurosci. (2023), 14 (8), 1459-1473 |
| 5-bromo-2-methylbenzo[*d*]thiazole | methyl iodide | 5-bromo-2,3-dimethylbenzo[*d*]thiazol-3-ium iodide | Liang, et al., Adv. Synth. Catal. (2024), 366 (1), 77-81 |
| 6-fluoro-2-methylbenzo[*d*]thiazole | methyl iodide | 6-fluoro-2,3-dimethylbenzo[*d*]thiazol-3-ium iodide | - |
| 5-fluoro-2-methylbenzo[*d*]thiazole | ethyl iodide | 3-ethyl-5-fluoro-2-methylbenzo[*d*]thiazol-3-ium iodide | WO2022/65354 |
| 5-fluoro-2-methylbenzo[*d*]thiazole | *n-*propyl iodide | 5-fluoro-2-methyl-3-propylbenzo[*d*]thiazol-3-ium iodide | - |
| 5-methoxy-2-methylbenzo[*d*]thiazole | methyl iodide | 5-methoxy-2,3-dimethyl benzo[*d*]thiazol-3-ium iodide | WO2009085226A2 |

For the synthesis of the compounds **1-23:** A solution of (hetero)aromatic aldehyde (0.3 mmol) and benzothiazolium precursor (0.3 mmol) in ethanol (5 mL) was heated at reflux (80 °C) for 9-38 h. After this time, the precipitate was filtered and washed with cold ethanol and diethyl ether to give the pure product.

As an example, the synthesis of (*E*)-2-(4-(Dimethylamino)styryl)-3-methylbenzo[*d*]thiazol-3-ium iodide (compound **1**).

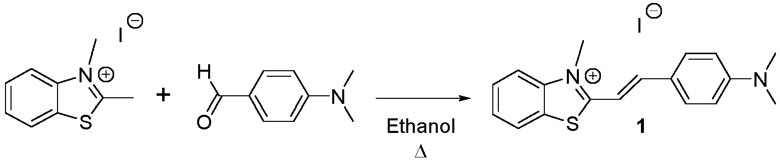

**Table 2. Commercial aldehyde reagents and benzothiazolium precursors for the synthesis of the compounds 1-23.**

| **(Hetero)aromatic aldehyde** | **Benzothiazolium salt** | **Final compounds** | **Ref.** |
|---|---|---|---|
| 4-(*N,N-*dimethylamino)benzaldehyde | 2,3-dimethylbenzo[*d*]thiazol-3-ium iodide | **1** | Coelho, et al., Dyes and Pigments (2015), 117, 163-169 |
| 4-(*N*,*N-*diphenylamino)benzaldehyde | 2,3-dimethylbenzo[*d*]thiazol-3-ium iodide | **2** | Sigmundová, et al., Collect. Czech. Chem. Commun. (2007), 72, 1069-1093 |
| 2,3,6,7-tetrahydro-1*H*,5*H-*pyrido[3,2,1-*ij*]quinoline-9-carbaldehyde | 2,3-dimethylbenzo[*d*]thiazol-3-ium iodide | **3** | Fülöpová et al., Journal of Molecular Structure (2012), 1027, 70-80 |
| 4-(*N*,*N-*diethylamino)benzaldehyde | 2,3-dimethylbenzo[*d*]thiazol-3-ium iodide | **4** | - |
| 4-(*N*,*N-*dimethylamino)benzaldehyde | 5-fluoro-2,3-dimethylbenzo[*d*]thiazol-3-ium iodide | **5** | US6001331 |
| 4-(*N*,*N-*dimethylamino)benzaldehyde | 5-chloro-2,3-dimethylbenzo[*d*]thiazol-3-ium iodide | **6** | US6001331 |
| 4-(*N*,*N-*dimethylamino)benzaldehyde | 2,3,5,6-tetramethylbenzo[*d*]thiazol-3-ium iodide | **7** | Mallesh, et al., ACS Chem. Neurosci. (2023), 14 (8), 1459-1473 |
| 4-(*N*,*N-*diethylamino)benzaldehyde | 5-fluoro-2,3-dimethylbenzo[*d*]thiazol-3-ium iodide | **8** | - |
| 4-(*N*,*N-*diethylamino)benzaldehyde | 5-chloro-2,3-dimethylbenzo[*d*]thiazol-3-ium iodide | **9** | - |
| 4-(*N*,*N-*diethylamino)benzaldehyde | 5-bromo-2,3-dimethylbenzo[*d*]thiazol-3-ium iodide | **10** | - |
| 4-(*N*,*N*-dimethylamino)-3-fluorobenzaldehyde | 5-fluoro-2,3-dimethylbenzo[*d*]thiazol-3-ium iodide | **11** | - |
| 4-(*N*,*N*-dimethylamino)-3-fluorobenzaldehyde | 6-fluoro-2,3-dimethylbenzo[*d*]thiazol-3-ium iodide | **12** | - |
| 4-(*N*,*N-*dimethylamino)benzaldehyde | 6-fluoro-2,3-dimethylbenzo[*d*]thiazol-3-ium iodide | **13** | - |
| 4-(*N*,*N-*dimethylamino)benzaldehyde | 3-ethyl-5-fluoro-2-methylbenzo[*d*]thiazol-3-ium iodide | **14** | - |
| 4-(*N*,*N-*diethylamino)benzaldehyde | 3-ethyl-5-fluoro-2-methylbenzo[*d*]thiazol-3-ium iodide | **15** | - |
| 4-(*N*,*N-*dimethylamino)benzaldehyde | 5-fluoro-2-methyl-3-propylbenzo[*d*]thiazol-3-ium iodide | **16** | - |
| 4-(*N*,*N-*diethylamino)benzaldehyde | 5-fluoro-2-methyl-3-propylbenzo[*d*]thiazol-3-ium iodide | **17** | - |
| 4-(*N*,*N-*dibutylamino)benzaldehyde | 2,3-dimethylbenzo[*d*]thiazol-3-ium iodide | **18** | - |
| 4-(*N*,*N-*dimethylamino)benzaldehyde | 5-methoxy-2-methyl-3-methylbenzo[*d*]thiazol-3-ium iodide | **19** | - |
| 4-(*N*,*N-*diphenylamino)benzaldehyde | 5-methoxy-2,3-dimethylbenzo[*d*]thiazol-3-ium iodide | **20** | - |
| 4-(*N*,*N-*diphenylamino)benzaldehyde | 5-fluoro-2,3-dimethylbenzo[*d*]thiazol-3-ium iodide | **21** | - |
| 4-(*N*,*N-*diphenylamino)benzaldehyde | 5-chloro-2,3-dimethylbenzo[*d*]thiazol-3-ium iodide | **22** | - |
| 4-(*N*,*N-*diphenylamino)benzaldehyde | 2,3,5,6-tetramethylbenzo[*d*]thiazol-3-ium iodide | **23** | - |

### Example 2. Characterization of the new benzothiazolium precursors and final compounds 1-23

NMR spectra were obtained on a Bruker Avance III 400 at an operating frequency of 400 MHz for ¹H and 100.6 MHz for ¹³C, using the solvent peak as an internal reference (*δ* relative to TMS). Peak assignments were supported by spin decoupling-double resonance and bidimensional heteronuclear techniques. High-resolution mass spectra (HRMS) were obtained on a QqTOF Impact IITM mass spectrometer (Bruker Daltonics) operating in ESI negative and positive modes.

### Benzothiazolium precursor. 6-fluoro-2,3-dimethylbenzo[d]thiazol-3-ium iodide

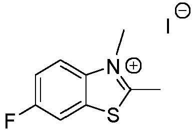

Colourless solid (reaction time = 22 h, yield = 78%). ¹H NMR (400 MHz, DMSO-*d₆*): *δ* = 3.14 (3H, s, CH₃), 4.18 (3H, s, N⁺CH₃), 7.83 (1H, dt, *J* = 2.8 and 9.2 Hz, H-5), 8.31 (1H, dd, *J* = 2.8 and 8.4 Hz, H-7), 8.34 (1H, dd, *J* = 4.4 and 9.2 Hz, H-4) ppm.

¹³C NMR (100.6 MHz, DMSO-*d₆*): *δ* = 17.26 (CH₃), 36.53 (N⁺CH₃), 110.88 (d, *J* = 28.67 Hz, C7), 117.93 (d, *J* = 25.75 Hz, C5), 118.78 (d, *J* = 9.86 Hz, C4), 130.26 (d, *J* = 12.37 Hz, C7a), 138.48 (C3a), 160.68 (d, *J* = 246.47 Hz, C6), 177.75 (C2) ppm. HRMS (ESI) *m*/*z:* [M-I⁻]⁺ calcd for C₉H₉NFS⁺ 182.0434, found 182.0435.

### Benzothiazolium precursor. 5-fluoro-2-methyl-3-propylbenzo[d]thiazol-3-ium iodide

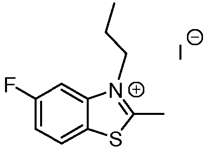

Off white solid (reaction time = 96 h, yield = 15%). ¹H NMR (400 MHz, DMSO-*d₆*): *δ* = 1.00 (3H, t, *J* = 7.2 Hz, N⁺CH₂CH₂CH₃), 1.81-1.90 (2H, sx, *J* = 7.6 Hz, N⁺CH₂CH₂CH₃), 3.20 (3H, s, CH₃), 4.62 (2H, t, *J* = 7.6 Hz, N⁺CH₂CH₂CH₃), 7.73 (1H, dt, *J* = 2.4 and 9.2 Hz, H-6), 8.41 (1H, dd, *J* = 2.4 and 9.6 Hz, H-4), 8.47 (1H, dd, *J* = 5.2 and 9.2 Hz, H-7) ppm.

¹³C NMR (100.6 MHz, DMSO-*d₆*): *δ* = 10.71 (N⁺CH₂CH₂CH₃), 17.16 (CH₃), 21.22 (N⁺CH₂CH₂CH₃), 50.74 (N⁺CH₂CH₂CH₃), 104.32 (d, *J* = 28.77 Hz, C4), 116.94 (d, *J* = 25.05 Hz, C6), 125.13 (d, *J* = 1.51 Hz, C7a), 126.65 (d, *J* = 10.26 Hz, C7), 142.12 (d, *J* = 12.47 Hz, C3a), 162.54 (d, *J* = 246.47 Hz, C5), 179.36 (C2) ppm.

HRMS (ESI) *m*/*z:* [M-I⁻]⁺ calcd for C₁₁H₁₃NFS⁺ 210.0753, found 210.0767.

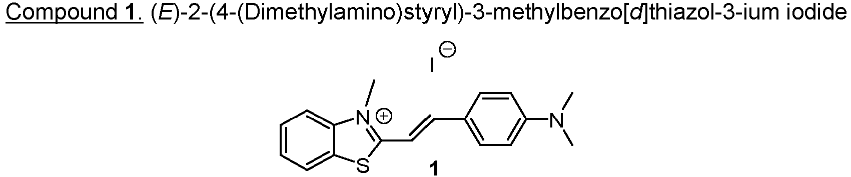

Dark brown solid (reaction time = 6 h, yield = 35%). ¹H NMR (400 Hz, DMSO-*d₆*): *δ* = 3.10 (6H, s, N(CH₃)₂), 4.22 (3H, s, N⁺CH₃), 6.63 (2H, d, *J* = 8.8 Hz, H-3' and H-5'), 7.62 (1H, d, *J* = 15.4 Hz, H-b), 7.67 (1H, dt, *J* = 0.9 and *J* = 7.8, H-6), 7.78 (1H, dt, *J* = 0.9 Hz and *J* = 7.8, H-5), 7.90 (2H, d, *J* = 8.8 Hz, H-2'- and H-6'), 8.06 (1H, d, *J* = 15.4 Hz, H-a), 8.07-8.09 (1H, m, H-7), 8.28 (1H, dd, *J* = 0.9 Hz and *J* = 8.0, H-4) ppm.

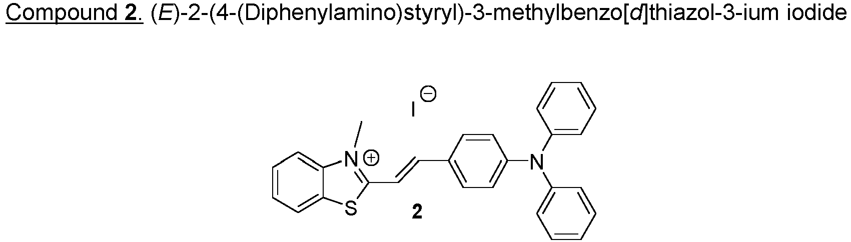

Dark red solid (reaction time = 6 h, yield = 46%). ¹H NMR (400 MHz, DMSO-*d₆*): *δ* = 4.25 (3H, s, N⁺CH₃), 6.88 (2H, d, *J* = 8.8 Hz, H-2' and H-6'), 7.17-7.24 (6H, m, 2x (H-2", H-4" and H-6")), 7.70-7.44 (4H, m, 2x (H-3" and H-5")), 7.71 (1H, dd, *J* = 0.8 Hz and 8.0 Hz, H-6), 7.76 (1H, d, *J* = 16 Hz, H-b), 7.81 (1H, dt, *J =* 1.2 Hz and 8.4 Hz, H-5), 7.90 (2H, d, 2H *J* = 8.8 Hz, H-3' and H-5'), 8.09 (1H, d, *J* = 16 Hz, H-a), 8.17 (1H, d, *J* = 8.4 Hz, H-7), 8.36 (1H, dd, *J* = 0.8 Hz and 8.0 Hz, H-4) ppm.

¹³C NMR (100.6 MHz, DMSO-*d₆*): *δ* = 36.16 (N⁺CH₃), 110.07 (Cb), 116.47 (C7), 118.92 (C2' and C6'), 124.03 (C4), 125.46 (C4"), 126.16 (C2", C6" and 4'), 127.34 (C7a), 127.99 (C6), 129.18 (C5), 130.02 (C3" and C5"), 131.86 (C3' and C5'), 141.96 (C3a), 145.46 (C1"), 148.54 (Ca), 151.33 (C1'), 171.64 (C2) ppm.

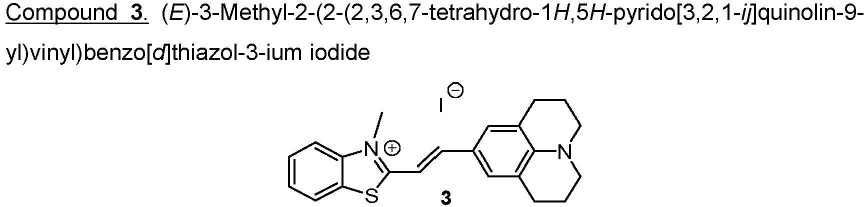

Dark blue solid (reaction time = 8 h, yield = 40%). ¹H NMR (400 MHz, DMSO-*d₆*): *δ* = 1.86-1.92 (4H, m, CH₂), 2.72 (4H, t, *J* = 6 Hz, CH₂), 3.37 (4H, *J* = 6 Hz, CH₂), 4.14 (3H, s, N⁺CH₃), 7.42 (1H, d, *J* = 16 Hz, H-a), 7.49 (2H, br s, H-2' and H-6'), 7.61 (1H, dt, *J* = 1.2 Hz and 7.2 Hz, H-5), 7.72 (1H, dt, *J* = 1.2 Hz and 7.2 Hz, H-6), 7.86 (1H, d, *J* = 14.8 Hz, H-b), 8.00 (1H, br d, *J* = 8.4 Hz, H-7), 8.21 (1H, br d, *J* = 8 Hz, H-4) ppm.

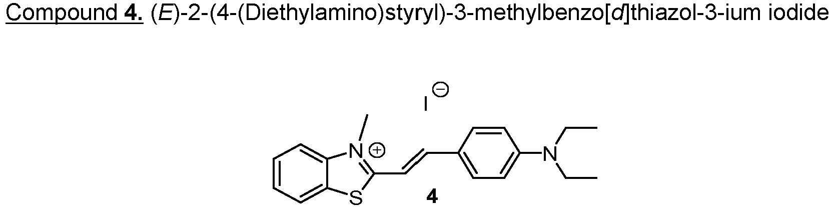

Dark purple solid (reaction time = 16 h, yield = 88%). ¹H NMR (400 MHz, DMSO-*d₆*): *δ* = 1.15 (6H, t, *J* = 6.8 Hz, N(CH₂CH₃)₂), 3.49 (4H, q, *J* = 6.8 Hz, N(CH₂-CH₃)₂), 4.20 (3H, s, N⁺CH₃), 6.81 (2H, d, *J* = 8.8 Hz, H-3' and H-5'), 7.56 (1H, d, *J* = 15.6 Hz, H-a), 7.66 (1H, dt, *J* = 1.2 Hz and 7.6 Hz, H-5), 7.76 (1H, dt, *J* = 1.2 Hz and 7.6 Hz, H-6), 7.82 (2H, d, *J =* 8.8 Hz, H-2' and H-6'), 8.02 (1H, d, *J* = 15.2 Hz, H-b), 8.06 (1H, d, *J* = 8.4 Hz, H-4), 8.25 (1H, dd, *J* = 0.8 Hz and 8 Hz, H-7) ppm.

¹³C NMR (100.6 MHz, DMSO-*d₆*): *δ* = 12.49 (N(CH₂CH₃)₂), 35.47 (N⁺CH₃), 44.15 (N(CH₂-CH₃)₂), 105.64 (Ca), 111.60 (C3' and C5'), 115.80 (C4), 121.01 (C1'), 123.72 (C7), 126.65 (C3a), 127.30 (C5), 128.78 (C6), 133.25 (C2' And C6'), 141.91 (C7a), 150.03 (Cb), 151.42 (C4'), 171.15 (C2) ppm.

HRMS (ESI) *m*/*z*: [M-I⁻]⁺ calcd for C₂₀H₂₃N₂S⁺ 323.1576, found 323.1584.

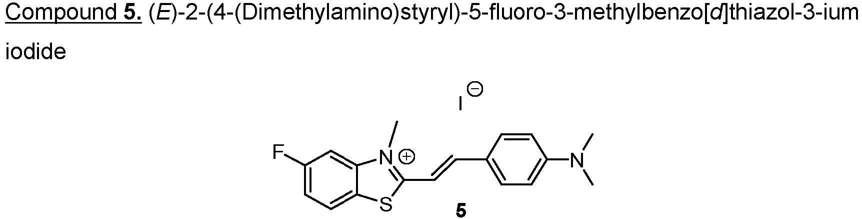

Dark purple solid (reaction time = 8 h, yield = 72%).¹H NMR (400 MHz, DMSO-*d₆*): *δ* = 3.10 (6H, s, N(CH₃)₂), 4.15 (3H, s, N⁺CH₃), 6.80 (2H, d, *J* = 9.2 Hz,H-3' and H-5'), 7.55 (1H, d, *J* = 15.2 Hz, H-a), 7.57 (1H, dt, *J* = 2.4 Hz and 9.2 Hz, H-6), 7.89 (2H, d, *J* = 9.2 Hz, H-2' and H-6'), 8.02 (1H, d, *J* = 15.2 Hz, H-b), 8.07 (1H, dd, *J* = 2.4 Hz and 9.6 Hz , H-4), 8.29 (1H, dd, *J* = 5.2 Hz and 8.8 Hz, H-7) ppm.

¹³C NMR (100.6 MHz, DMSO-*d₆*): *δ* = 35.78 (N⁺CH₃), 39.76 (N(CH₃)₂), 103.45 (d, *J* = 29.17 Hz, C4), 106.07 (Ca), 111.97 (C3' and C5'), 115.40 (d, *J* = 25.15 Hz, C6), 121.41 (C1'), 122.55 (C7a), 125.54 (d, *J* = 10.06 Hz, C7), 133.03 (C2' and C6'), 143.22 (d, *J* = 12.07 Hz, C3a), 150.43 (Cb), 153.62 (C4'), 162.33 (d, *J* = 245.46 Hz, C5), 173.08 (C2) ppm

HRMS (ESI) *m*/*z*: [M-I⁻]⁺ calcd for C₁₈H₁₈FN₂S⁺ 313.1169, found 313.1178.

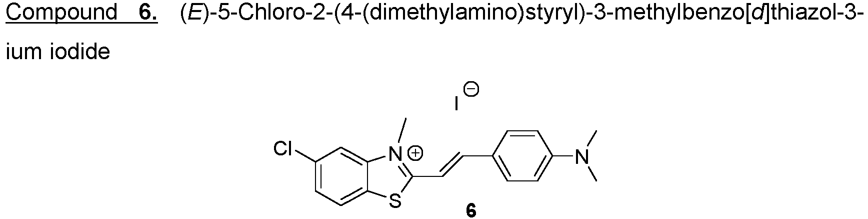

Dark purple solid (reaction time = 9 h, yield = 85%). ¹H NMR (400 MHz, DMSO-*d₆*): *δ* = 3.09 (6H, s, N(CH₃)₂), 4.16 (3H, s, N⁺CH₃), 6.80 (2H, d, *J* = 9.2 Hz, H-3' and H-5'), 7.55 (1H, d, *J* = 15.6 Hz, H-a), 7.71 (1H, dd, *J* = 2 and 8.8 Hz, H-6), 7.89 (2H, d, *J* = 9.2 Hz, H-2' and H-6'), 8.05 (1H, d, *J* = 15.2 Hz, H-b), 8.25 (1H, s, H-4), 8.27 (1H, d, *J* = 8.4 Hz, H-7) ppm.

¹³C NMR (100.6 MHz, DMSO-*d₆*): *δ* = 35.69 (N⁺CH₃), 39.76 (N(CH₃)₂), 105.87 (Ca), 111.96 (C3' and C5'), 115.82 (C4), 121.41 (C1'), 125.16 (C7), 125.62 (C7a), 127.27 (C6), 133.10 (C2' and C6'), 133.71 (C5), 142.99 (C3a), 150.78 (Cb), 153.67 (C4'), 172.46 (C2) ppm.

HRMS (ESI) *m*/*z*: [M-I⁻]⁺ calcd for C₁₈H₁₈CIN₂S⁺ 329.0874, found 329.0876.

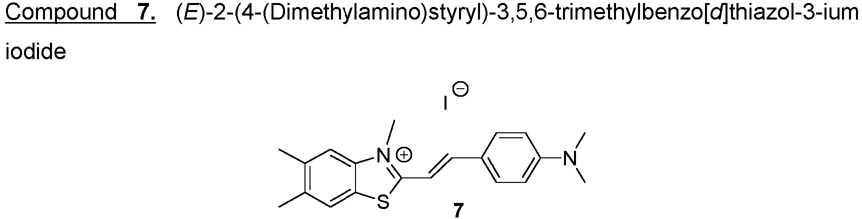

Dark purple solid (reaction time = 36 h, yield = 49%). ¹H NMR (400 MHz, DMSO-*d₆*): *δ* = 2.39 (3H, s, Ar-CH₃), 2.43 (3H, s, Ar-CH₃), 3.09 (6H, s, N(CH₃)₂), 4.18 (3H, s, N⁺CH₃), 6.82 (2H, d, *J* = 8 Hz, H-3' and H-5'), 7.57 (1H, d, *J* = 15.2 Hz, H-a), 7.86 (2H, d, *J* = 9.2 Hz, H-2' and H-6'), 7.91 (1H, s, H-4), 7.99 (1H, d, *J* = 15.2 Hz, H-b), 8.02 (1H, s, H-7) ppm.

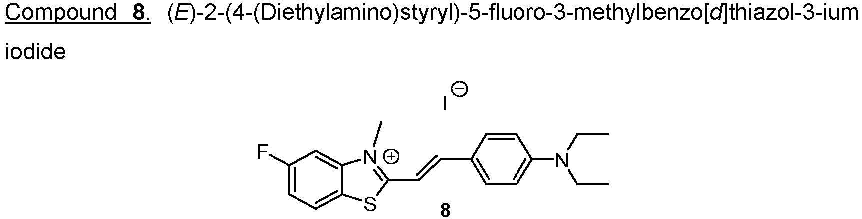

Dark purple solid (reaction time = 8 h, yield = 80%). ¹H NMR (400 MHz, DMSO-*d₆*): *δ* = 1.15 (6H, t, *J* = 6.8 Hz, N(CH₂CH₃)₂), 3.50 (4H, q, *J* = 6.8 Hz, N(CH₂CH₃)₂), 4.15 (3H, N⁺CH₃), 6.83 (2H, d, *J* = 9.2 Hz, H-3' and H-5'), 7.54 (1H, d, *J* = 15.2 Hz, H-a), 7.59 (1H, dt, *J* = 2.4 and 8.8 Hz, H-6), 7.88 (2H, d, *J* = 9.2 Hz, H-2' and H-6'), 8.04 (1H, d, *J* = 15.2 Hz, H-b), 8.08 (1H, dd, *J* = 2.4 and 9.6 Hz , H-4), 8.30 (1H, dd, *J* = 5.2 and 8.8 Hz, H-7) ppm.

¹³C NMR (100.6 MHz, DMSO-*d₆*): *δ* = 12.54 (N(CH₂CH₃)₂), 35.78 (N⁺CH₃), 44.26 (N(CH₂CH₃)₂), 103.54 (d, *J* = 28.17 Hz, C4), 105.51 (Ca), 111.72 (C3' and C5'), 115.48 (d, *J* = 24.14 Hz, C6), 121.06 (C1'), 122.44 (C7a), 125.53 (d, *J* = 10.06 Hz, C7), 133.53 (C2' and C6'), 143.31 (d, *J* = 13.08 Hz, C3a), 150.40 (Cb), 151.68 (C4'), 162.3 (d, *J* = 244.46 Hz, C5), 172.94 (C2) ppm.

HRMS (ESI) *m*/*z:* [M-I⁻]⁺ calcd for C₂₀H₂₂FN₂S⁺ 341.1482, found 341.1495.

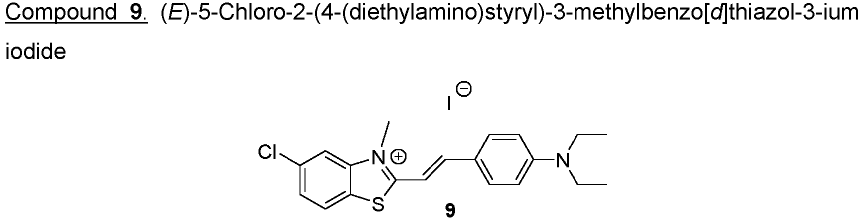

Dark green solid (reaction time = 8 h, yield = 83%). ¹H NMR (400 MHz, DMSO-*d₆*): *δ* = 1.15 (6H, t, *J* = 6.8 Hz, N(CH₂CH₃)₂), 3.49 (4H, q, *J* =6.8 Hz, N(CH₂CH₃)₂), 4.15 (3H, N⁺CH₃), 6.80 (2H, d, *J* = 9.2 Hz, H-3' and H-5'), 7.51 (1H, d, *J* = 15.2 Hz, H-a), 7.69 (1H, dd, *J =* 2 and 8.8 Hz, H-6), 7.87 (2H, d, *J* = 8.8 Hz, H-2' and H-6'), 8.03 (1H, d, *J* = 15.2 Hz, H-b), 8.23 (1H, d, *J* = 2 Hz, H-4), 8.26 (1H, d, *J* = 8.8 Hz, H-7) ppm.

¹³C NMR (100.6 MHz, DMSO-*d₆*): *δ* = 12.52 (N(CH₂CH₃)₂), 35.63 (N⁺CH₃), 44.26 (N(CH₂CH₃)₂), 105.31 (Ca), 111.72 (C3' and C5'), 115.75 (C4), 121.07 (C1'), 125.11 (C7), 125.52 (C7a), 127.19 (C6), 133.61 (C2' and C6'), 133.69 (C5), 143.03 (C3a), 150.73 (Cb), 151.73 (C4'), 172.29 (C2) ppm.

HRMS (ESI) *m*/*z:* [M-I⁻]⁺ calcd for C₂₀H₂₂CIN₂S⁺ 357.1187, found 357.1191.

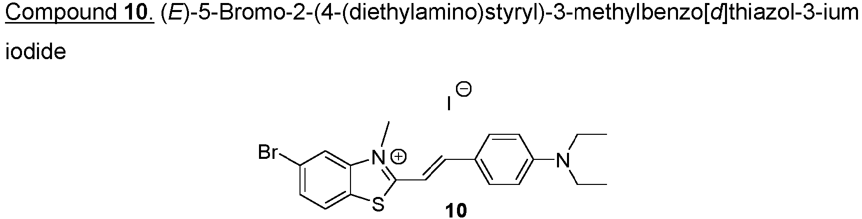

Dark purple solid (reaction time = 9 h, yield = 78%). ¹H NMR (400 MHz, DMSO-*d₆*): *δ* = 1.15 (6H, t, *J* = 6.8 Hz, N(CH₂CH₃)₂), 3.50 (4H, q, *J* = 6.8 Hz, N(CH₂CH₃)₂), 4.15 (3H, N⁺CH₃), 6.82 (2H, d, *J* = 9.2 Hz, H-3' and H-5'), 7.52 (1H, d, *J* = 15.2 Hz, H-a), 7.82 (1H, dd, *J* = 2 and 8.8 Hz, H-6), 7.87 (2H, d, *J* = 9.2 Hz, H-2' and H-6'), 8.05 (1H, d, *J* = 15.2 Hz, H-b), 8.19 (1H, d, *J* = 8.4 Hz, H-7), 8.27 (1H, d, *J* = 1.6 Hz, H-4) ppm. ¹³C NMR (100.6 MHz, DMSO-*d₆*): *δ* = 12.55 (N(CH₂CH₃)₂), 35.63 (N⁺CH₃), 44.30 (N(CH₂CH₃)₂), 105.30 (Ca), 111.76 (C3' and C5'), 118.57 (C4), 121.11 (C1'), 121.79 (C7a), 125.32 (C7), 126.01 (C5), 129.97 (C6), 133.63 (C2' and C6'), 143.22 (C3a), 150.79 (Cb), 151.77 (C4'), 172.06 (C2) ppm.

HRMS (ESI) *m*/*z*: [M-I⁻]⁺ calcd for C₂₀H₂₂N₂SBr⁺ 401.0687, found 401.0682.

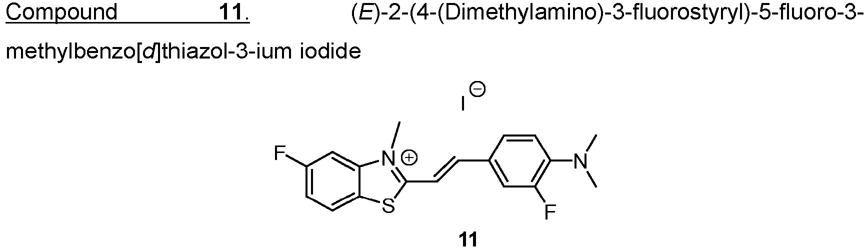

Dark brown solid (reaction time = 10 h, yield = 72%). ¹H NMR (400 MHz, DMSO-*d₆*): *δ* = 3.06 (6H, d, *J* = 2 Hz, N(CH₃)₂), 4.23 (3H, s, N⁺CH₃), 6.97 (1H, t, *J* = 9.2 Hz, H-5'), 7.65 (1H, dt, *J* = 2.4 and 8.8 Hz, H-6), 7.70 (1H, dd, *J* = 2 and 8.8 Hz, H-6'), 7.74 (1H, d, *J* = 15.6 Hz, H-a), 7.95 (1H, dd, *J* = 2 and 16 Hz, H-2'), 8.11 (1H, d, *J* = 15.6 Hz, H-b), 8.18 (1H, dd, *J* = 2.4 and 9.6 Hz, H-4), 8.39 (1H, dd, *J* = 5.2 and 8.8 Hz, H-7) ppm.

¹³C NMR (100.6 MHz, DMSO-*d₆*): *δ* = 36.23 (N⁺CH₃), 41.94 (d, *J =* 4.02 Hz, N(CH₃)₂), 103.86 (d, *J* = 29.17 Hz, C4), 109.65 (Ca), 116.18 (d, *J =* 24.95 Hz, C6), 116.57 (d, *J* = 8.75 Hz, C5'), 116.66 (d, *J* = 18.21 Hz, C2'), 123.15 (d, *J* = 2.01 Hz, C7a), 124.18 (d, *J* = 8.05 Hz, C1'), 125.88 (d, *J* = 10.06 Hz, C7), 129.33 (C6'), 143.22 (d, *J* = 13.08 Hz, C3a), 143.50 (d, *J* = 8.05 Hz, C4'), 148.52 (d, *J* = 3.02 Hz, Cb), 151.92 (d, *J* = 243.45 Hz, C3'), 162.40 (d, *J* = 246.47 Hz, C5), 173.46 (C2) ppm.

HRMS (ESI) *m*/*z*: [M-I⁻]⁺ calcd for C₁₈H₁₇F₂N₂S⁺ 331.1075, found 331.1084.

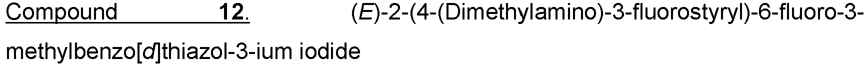

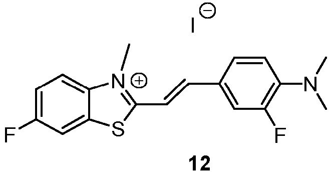

Purple solid (reaction time = 10 h, yield = 52%). ¹H NMR (400 MHz, DMSO-*d₆*): *δ* = 3.05 (6H, d, *J* = 2 Hz, N(CH₃)₂), 4.26 (3H, s, N⁺CH₃), 6.97 (1H, t, *J* = 9.2 Hz, H-5'), 7.69 (1H, dd, *J =* 2 and 8.8 Hz, H-6'), 7.74 (1H, dt, *J* = 2.8 and 9.2 Hz, H-5), 7.75 (1H, d, *J =* 15.6 Hz, H-a), 7.95 (1H, dd, *J* = 2 and 16 Hz, H-2'), 8.09 (1H, d, *J =* 15.6 Hz, H-b), 8.20 (1H, dd, *J* = 4.4 and 9.6 Hz, H-4), 8.29 (1H, dd, *J* = 2.4 and 8.4 Hz, H-7) ppm.

¹³C NMR (100.6 MHz, DMSO-*d₆*): *δ* = 36.33 (N⁺CH₃), 41.94 (d, *J =* 6.04 Hz, N(CH₃)₂), 109.74 (Ca), 110.69 (d, *J =* 28. 77 Hz, C7), 116.52 (d, *J =* 15.09 Hz, C2'), 116.65 (d, *J* = 2.41 Hz, C5'), 117.50 (d, *J* = 25.25 Hz, C5), 118.25 (d, *J* = 9.66 Hz, C4), 124.25 (d, *J* = 8.05 Hz, C1'), 128.93 (d, *J* = 13.08 Hz, C7a), 129.20 (C6'), 138.87 (C3a), 143.43 (d, *J* = 2.01 Hz, C4'), 148.43 (d, *J* = 2.01 Hz, Cb), 151.97 (d, *J* = 243.45 Hz, C3'), 160.72 (d, *J =* 247.48 Hz, C6), 171.80 (C2) ppm.

HRMS (ESI) *m*/*z:* [M-I⁻]⁺ calcd for C₁₈H₁₇N₂F₂S⁺ 331.1081, found 331.1135.

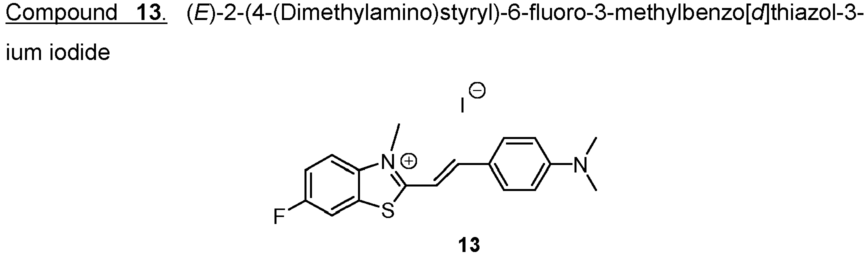

Dark purple solid (reaction time = 10 h, yield = 83%). ¹H NMR (400 MHz, DMSO-*d₆*): *δ* = 3.11 (6H, s, N(CH₃)₂), 4.20 (3H, s, N⁺CH₃), 6.84 (2H, d, *J =* 9.2 Hz, H-3' and H-5'), 7.58 (1H, d, *J =* 15.2 Hz, H-a), 7.69 (1H, dt, *J* = 2.4 and 8.8 Hz, H-5), 7.90 (2H, d, *J =* 9.2 Hz, H-2' and H-6'), 8.05 (1H, d, *J =* 15.2 Hz, H-b), 8.12 (1H, dd, *J =* 4.4 and 9.2 Hz , H-4), 8.23 (1H, dd, *J* = 2.8 and 8.4 Hz, H-7) ppm.

¹³C NMR (100.6 MHz, DMSO-*d₆*): *δ* = 35.85 (N⁺CH₃), 39.71 (N(CH₃)₂), 106.19 (Ca), 110.50 (d, *J =* 28.17 Hz, C7), 111.92 (C3' and C5'), 117.00 (d, *J =* 25.15 Hz, C5), 117.62 (d, *J =* 10.06 Hz, C4), 121.42 (C1'), 128.40 (d, *J =* 12.07 Hz, C7a), 132.86 (C2' and C6'), 138.81 (d, *J =* 1.00 Hz, C3a), 150.31 (Cb), 153.51 (C4'), 160.46 (d, *J =* 246.47 Hz, C6), 171.52 (C2) ppm.

HRMS (ESI) *m*/*z*: [M-I⁻]⁺ calcd for C₁₈H₁₈N₂FS⁺ 313.1175, found 313.1224.

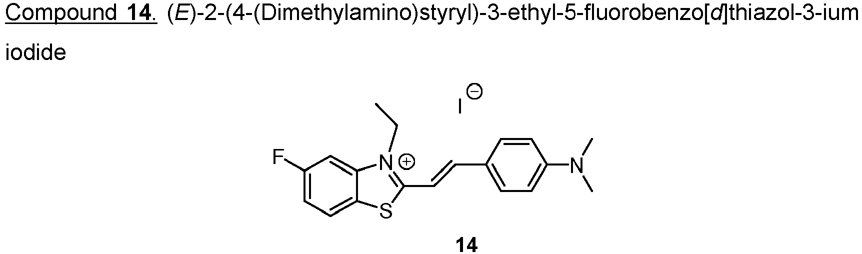

Purple solid (reaction time = 10 h, yield = 85%). ¹H NMR (400 MHz, DMSO-*d₆*): *δ* = 1.40 (3H, t, *J* = 7.2 Hz, N⁺CH₂CH₃), 3.12 (6H, s, N(CH₃)₂), 4.77 (2H, q, *J* = 7.2 Hz, N⁺CH₂CH₃), 6.84 (2H, d, *J* = 9.2 Hz,H-3' and H-5'), 7.58 (1H, d, *J* = 15.2 Hz, H-a), 7.59 (1H, dt, *J* = 2.4 and 9.2 Hz, H-6), 7.92 (2H, d, *J* = 9.2 Hz, H-2' and H-6'), 8.09 (1H, d, *J =* 15.2 Hz, H-b), 8.16 (1H, dd, *J* = 2.4 and 9.6 Hz , H-4), 8.33 (1H, dd, *J* = 5.2 and 8.8 Hz, H-7) ppm.

¹³C NMR (100.6 MHz, DMSO-*d₆*): *δ* = 13.91 (N⁺CH₂CH₃), 39.71 (N(CH₃)₂), 43.71 (N⁺CH₂CH₃), 103.21 (d, *J =* 29.17 Hz, C4), 105.39 (Ca), 111.94 (C3' and C5'), 115.55 (d, *J =* 25.15 Hz, C6), 121.41 (C1'), 122.94 (C7a), 125.70 (d, *J =* 9.05 Hz, C7), 133.22 (C2' and C6'), 142.11 (d, *J =* 13.08 Hz, C3a), 150.98 (Cb), 153.69 (C4'), 162.30 (d, *J* = 245.46 Hz, C5), 172.94 (C2) ppm

HRMS (ESI) *m*/*z*: [M-I⁻]⁺ calcd for C₁₉H₂₀FN₂S⁺ 327.1326, found 327.1337.

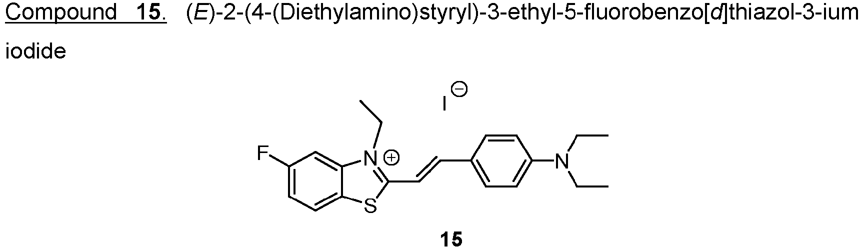

Dark purple solid (reaction time = 10 h, yield = 87%). ¹H NMR (400 MHz, DMSO-*d₆*): *δ* = 1.16 (6H, t, *J* = 7.2 Hz, N(CH₂CH₃)₂), 1.39 (3H, t, *J* = 7.2 Hz, N⁺CH₂CH₃), 3.51 (4H, q, *J* = 6.8 Hz, N(CH₂CH₃)₂), 4.77 (3H, N⁺CH₂CH₃), 6.83 (2H, d, *J* = 9.2 Hz,H-3' and H-5'), 7.54 (1H, d, *J* = 15.2 Hz, H-a), 7.57 (1H, dt, *J* = 2.4 and 9.2 Hz, H-6), 7.90 (2H, d, *J =* 9.2 Hz, H-2' and H-6'), 8.07 (1H, d, *J =* 15.2 Hz, H-b), 8.14 (1H, dd, *J* = 2.4 and 9.6 Hz , H-4), 8.31 (1H, dd, *J* = 5.2 and 9.2 Hz, H-7) ppm.

¹³C NMR (100.6 MHz, DMSO-*d₆*): *δ* = 12.50 (N(CH₂CH₃)₂), 13.64 (N⁺CH₂CH₃), 43.61 (N⁺CH₂CH₃), 44.25 (N(CH₂CH₃)₂), 103.15 (d, *J =* 29.17 Hz, C4), 104.84 (Ca), 111.69 (C3' and C5'), 115.40 (d, *J =* 24.14 Hz, C6), 121.05 (C1'), 122.82 (C7a), 125.65 (d, *J* = 10.06 Hz, C7), 133.69 (C2' and C6'), 142.12 (d, *J =* 12.07 Hz, C3a), 150.92 (Cb), 151.71 (C4'), 162.30 (d, *J =* 245.46 Hz, C5), 172.39 (C2) ppm

HRMS (ESI) *m*/*z*: [M-I⁻]⁺ calcd for C₂₁H₂₄FN₂S⁺ 355.1639, found 355.1646.

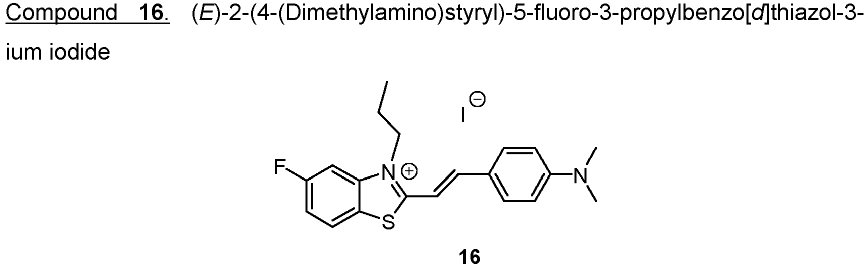

Purple solid (reaction time = 10 h, yield = 83%). ¹H NMR (400 MHz, DMSO-*d₆*): *δ* = 0.99 (3H, t, *J* = 7.2 Hz, N⁺CH₂CH₂CH₃), 1.82 (3H, sx, *J* = 7.2 Hz, N⁺CH₂CH₂CH₃), 3.11 (6H, s, N(CH₃)₂), 4.71 (2H, t, *J* = 7.2 Hz, N⁺CH₂CH₂CH₃), 6.84 (2H, d, *J* = 9.2 Hz, H-3' and H-5'), 7.57 (1H, d, *J* = 15.2 Hz, H-a), 7.58 (1H, dt, *J* = 2.4 and 8.8 Hz, H-6), 7.92 (2H, d, *J* = 8.8 Hz, H-2' and H-6'), 8.09 (1H, d, *J* = 15.2 Hz, H-b), 8.18 (1H, dd, *J* = 2.4 and 9.6 Hz , H-4), 8.32 (1H, dd, *J* = 5.2 and 9.2 Hz, H-7) ppm.

¹³C NMR (100.6 MHz, DMSO-*d₆*): *δ* = 10.63 (N⁺CH₂CH₂CH₃), 21.76 (N⁺CH₂CH₂CH₃), 39.80 (N(CH₃)₂), 49.29 (N⁺CH₂CH₂CH₃), 103.48 (d, *J =* 29.17 Hz, C4), 105.65 (Ca), 112.00 (C3' and C5'), 115.60 (d, *J* = 25.15 Hz, C6), 121.42 (C1'), 122.81 (C7a), 125.67 (d, *J* = 10.06 Hz, C7), 133.24 (C2' and C6'), 142.52 (d, *J* = 12.07 Hz, C3a), 150.92 (Cb), 153.73 (C4'), 162.47 (d, *J* = 245.46 Hz, C5), 172.98 (C2) ppm.

HRMS (ESI) *m*/*z*: [M-I⁻]⁺ calcd for C₂₀H₂₂FN₂S⁺ 341.1482, found 341.1481.

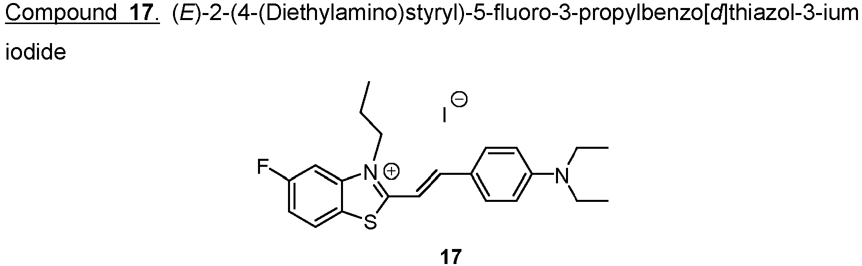

Dark purple solid (reaction time = 10 h, yield = 78%). ¹H NMR (400 MHz, DMSO-*d₆*): *δ* = 0.99 (3H, t, *J* = 7.2 Hz, N⁺CH₂CH₂CH₃), 1.15 (6H, t, *J* = 6.8 Hz, N(CH₂CH₃)₂), 1.81 (3H, sx, *J* = 7.2 Hz, N⁺CH₂CH₂CH₃), 3.51 (4H, q, *J* = 6.8 Hz, N(CH₂CH₃)₂), 4.69 (2H, t, *J =* 7.6 Hz, N⁺CH₂CH₂CH₃), 6.83 (2H, d, *J* = 9.2 Hz, H-3' and H-5'), 7.54 (1H, d, *J* = 15.2 Hz, H-a), 7.56 (1H, dt, *J* = 2.4 and 9.2 Hz, H-6), 7.90 (2H, d, *J* = 8.8 Hz, H-2' and H-6'), 8.06 (1H, d, *J* = 15.2 Hz, H-b), 8.17 (1H, dd, *J* = 2.4 and 10 Hz , H-4), 8.31 (1H, dd, *J* = 5.2 and 9.2 Hz, H-7) ppm.

¹³C NMR (100.6 MHz, DMSO-*d₆*): *δ* = 10.61 (N⁺CH₂CH₂CH₃), 12.50 (N(CH₂CH₃)₂), 21.69 (N⁺CH₂CH₂CH₃), 44.27 (N(CH₂CH₃)₂), 49.18 (N⁺CH₂CH₂CH₃), 103.38 (d, *J* = 28.17 Hz, C4), 105.09 (Ca), 111.71 (C3' and C5'), 115.45 (d, *J* = 25.15 Hz, C6), 121.04 (C1'), 122.68 (C7a), 125.61 (d, *J* = 10.06 Hz, C7), 133.69 (C2' and C6'), 142.52 (d, *J* = 12.07 Hz, C3a), 150.82 (Cb), 151.73 (C4'), 162.45 (d, *J =* 244.46 Hz, C5), 172.77 (C2) ppm.

HRMS (ESI) *m*/*z*: [M-I⁻]⁺ calcd for C₂₂H₂₆N₂FS⁺ 369.1801, found 369.1835.

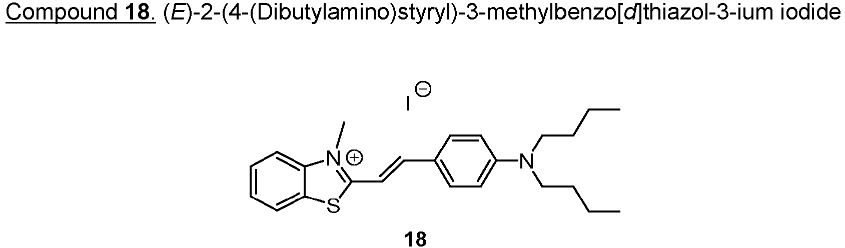

Dark purple solid (reaction time = 10 h, yield = 66%). ¹H NMR (400 MHz, DMSO-*d₆*): *δ* = 0.93 (6H, t, *J* = 7.2 Hz, N(CH₂CH₂CH₂CH₃)₂), 1.35 (4H, sx, *J* = 7.6 Hz, N(CH₂CH₂CH₂CH₃)₂), 1.55 (4H, qu, *J =* 7.2 Hz, N(CH₂CH₂CH₂CH₃)₂), 3.43 (4H, t, *J* = 7.6 Hz, N(CH₂CH₂CH₂CH₃)₂), 4.20 (3H, s, N⁺CH₃), 6.79 (2H, d, *J* = 8.8 Hz, H-3' and H-5'), 7.56 (1H, d, *J =* 15.2 Hz, H-a), 7.66 (1H, dt, *J =* 1.2 and 7.6 Hz, H-5), 7.76 (1H, dt, *J* = 1.2 and 7.6 Hz, H-6), 7.86 (2H, d, *J* = 8.8 Hz, H-2' and H-6'), 8.02 (1H, d, *J =* 15.2 Hz, H-b), 8.06 (1H, d, *J =* 8 Hz, H-4), 8.27 (1H, dd, *J* = 0.8 and 8 Hz, H-7) ppm. ¹³C NMR (100.6 MHz, DMSO-*d₆*): *δ* = 13.81 (N(CH₂CH₂CH₂CH₃)₂), 19.54 (N(CH₂CH₂CH₂CH₃)₂), 29.07 (N(CH₂CH₂CH₂CH₃)₂)), 35.46 (N⁺CH₃), 49.95 (N(CH₂CH₂CH₂CH₃)₂), 105.64 (Ca), 111.74 (C3' and C5'), 115.81 (C4), 121.00 (C1'), 123.72 (C7), 126.66 (C3a), 127.31 (C5), 128.79 (C6), 133.14 (C2' and C6'), 141.91 (C7a), 149.98 (Cb), 151.83 (C4'), 171.10 (C2) ppm.

HRMS (ESI) *m*/*z*: [M-I⁻]⁺ calcd for C₂₄H₃₁N₂S⁺ 379.2202, found 379.2212.

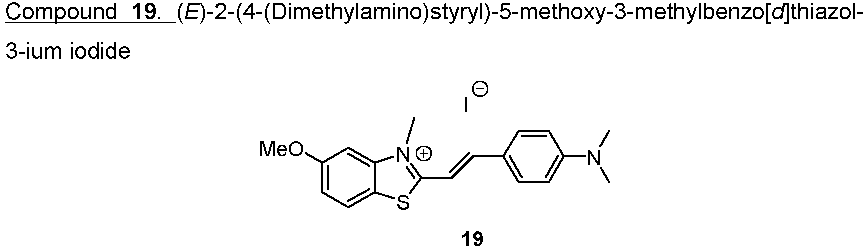

Dark brown solid (reaction time = 36 h, yield = 64%). ¹H NMR (400 MHz, DMSO-*d₆*): *δ* = 3.09 (6H, s, N(CH₃)₂), 3.93 (3H, s, OCH₃), 4.20 (3H, s, N⁺CH₃), 6.80 (2H, d, *J* = 9.2 Hz, H-3' and H-5'), 7.29 (1H, dd, *J* = 2.4 and 8.8 Hz, H-6), 7.58 (1H, d, *J* = 15.2 Hz, H-a), 7.61 (1H, s, H-4), 7.87 (2H, d, *J* = 8.8 Hz, H-2' and H-6'), 7.99 (1H, d, *J* = 15.2 Hz, H-b), 8.15 (1H, d, *J* = 9.2 Hz, H-7) ppm.

¹³C NMR (100.6 MHz, DMSO-*d₆*): *δ* = 35.69 (N⁺CH₃), 39.71 (N(CH₃)₂), 56.30 (OCH₃), 99.84 (C4), 106.54 (Ca), 111.91 (C3' and C5'), 116.48 (C6), 118.37 (C7a), 121.44 (C1'), 124.44 (C7), 132.58 (C2' and C6'), 143.38 (C3a), 149.09 (Cb), 153.33 (C4'), 160.44 (C5), 171.84 (C2) ppm.

HRMS (ESI) *m*/*z*: [M-I⁻]⁺ calcd for C₁₉H₂₁N₂OS⁺ 325.1369, found 325.1376.

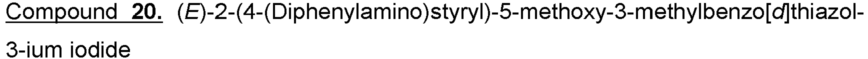

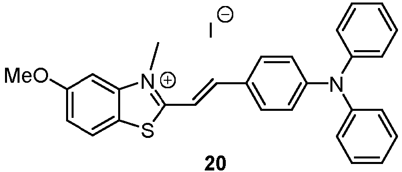

Dark red solid (reaction time = 36 h, yield = 43%).¹H NMR (400 MHz, DMSO-*d₆*): *δ* = 3.93 (3H, s, OCH₃), 4.26 (3H, s, N⁺CH₃), 6.89 (2H, d, *J =* 9.2 Hz, H-3' and H-5'), 7.18 (4H, d, *J* = 7.2 Hz, (H-2"and H-6") x 2), 7.22 (2H, t, *J* = 7.2 Hz, H-4" x 2), 7.36 (1H, dd, *J* = 2.4 and 8.8 Hz, H-6), 7.42 (4H, t, *J* = 7.2 Hz, (H-3" and H-5") x 2), 7.68 (1H, s, H-4), 7.73 (1H, d, *J* = 15.6 Hz, H-a), 7.87 (2H, d, *J =* 8.8 Hz, H-2' and H-6'), 8.04 (1H, d, *J* = 15.6 Hz, H-b), 8.22 (1H, d, *J* = 9.2 Hz, H-7) ppm.

¹³C NMR (100.6 MHz, DMSO-*d₆*): *δ* = 36.08 (N⁺CH₃), 56.37 (OCH₃), 100.00 (C4), 110.36 (Ca), 117.40 (C6), 119.11 (C3', C5', and C7a), 124.71 (C7), 125.39 (C4" × 2), 126.10 ((C2" and C6") x 2), 126.27 (C1'), 130.01 ((C3" and C5") x 2), 131.63 (C2' and C6'), 143.50 (C3a), 145.55 (C1" x 2), 147.51 (Cb), 151.18 (C4'), 160.64 (C5), 172.00 (C2) ppm.

HRMS (ESI) *m*/*z:* [M-I⁻]⁺ calcd for C₂₉H₂₅N₂OS⁺ 449.1682, found 449.1690.

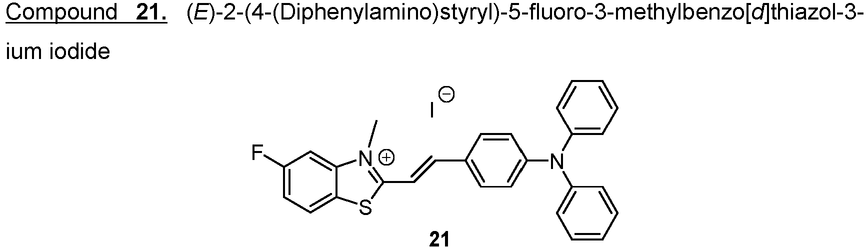

Dark green solid (reaction time = 9 h, yield = 51%).¹H NMR (400 MHz, DMSO-*d₆*): *δ* = 4.23 (3H, s, N⁺CH₃), 6.89 (2H, d, *J* = 8.8 Hz, H-3' and H-5'), 7.20 (4H, d, *J* = 7.6 Hz, (H-2"and H-6") x 2), 7.24 (2H, t, *J* = 7.4 Hz, H-4" x 2), 7.43 (4H, t, *J* = 7.6 Hz, (H-3" and H-5") × 2), 7.66 (1H, dt, *J* = 2.4 and 8.8 Hz, H-6), 7.73 (1H, d, *J* = 15.6 Hz, H-a), 7.90 (2H, d, *J* = 8.8 Hz, H-2' and H-6'), 8.12 (1H, d, *J* = 15.6 Hz, H-b), 8.20 (1H, dd, *J* = 2.4 and 9.6, H-4), 8.40 (1H, dd, *J* = 5.2 and 9.2 Hz, H-7) ppm.

¹³C NMR (100.6 MHz, DMSO-*d₆*): *δ* = 36.27 (N⁺CH₃), 103.94 (d, *J* = 28.17 Hz, C4), 109.98 (Ca), 116.31 (d, *J* = 25.15 Hz, C6), 118.85 (C3' and C5'), 123.23 (C7a), 125.55 (C4" x 2), 125.98 (d, *J* = 9.96 Hz, C7), 126.05 (C1'), 126.22 ((C2" and C6") x 2), 130.03 ((C3" and C5") x 2), 131.99 (C2' and C6'), 143.27 (d, *J* = 12.07 Hz, C3a), 145.41 (C1" x 2), 148.94 (Cb), 151.52 (C4'), 162.42 (d, *J =* 245.46 Hz, C5), 173.65 (C2) ppm.

HRMS (ESI) *m*/*z*: [M-I⁻]⁺ calcd for C₂₈H₂₂FN₂S⁺ 437.1482, found 437.1493.

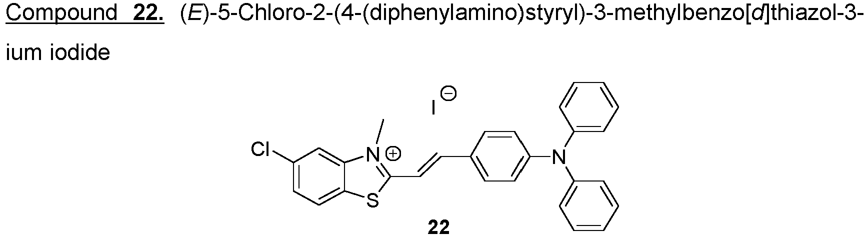

Dark red solid (reaction time = 10 h, yield = 56%). : ¹H NMR (400 MHz, DMSO-*d₆*): *δ* = 4.24 (3H, s, N⁺CH₃), 6.88 (2H, d, *J =* 9.2 Hz, H-3' and H-5'), 7.20 (4H, d, *J =* 7.6 Hz, (H-2"and H-6") x 2), 7.24 (2H, t, *J* = 7.4 Hz, H-4" x 2 ), 7.43 (4H, t, *J* = 7.6 Hz, (H-3" and H-5") x2), 7.73 (1H, d, *J =* 15.6 Hz, H-a), 7.79 (1H, dd, *J =* 2.4 and 8.4 Hz, H-6), 7.90 (2H, d, *J =* 9.2 Hz, H-2' and H-6'), 8.13 (1H, d, *J =* 15.6 Hz, H-b), 8.37 (1H, d, *J* = 8.4, H-7), 8.38 (1H, d, *J =* 1.6 Hz H-4) ppm.

¹³C NMR (100.6 MHz, DMSO-*d₆*): *δ* = 36.22 (N⁺CH₃), 109.82 (Ca), 116.46 (C4), 118.78 (C3' and C5'), 125.49 (C7), 125.60 (C4" x 2), 126.01 (C7a and C1'), 126.26 ((C2" and C6") x 2), 128.01 (C6), 130.04 ((C3" and C5") x 2), 132.07 (C2' and C6'), 134.09 (C5), 143.06 (C3a), 145.38 (C1" x 2), 149.34 (Cb), 151.60 (C4'), 173.13 (C2) ppm.

HRMS (ESI) *m*/*z:* [M-I⁻]⁺ calcd for C₂₈H₂₂ClN₂S⁺ 453.1187, found 453.1193.

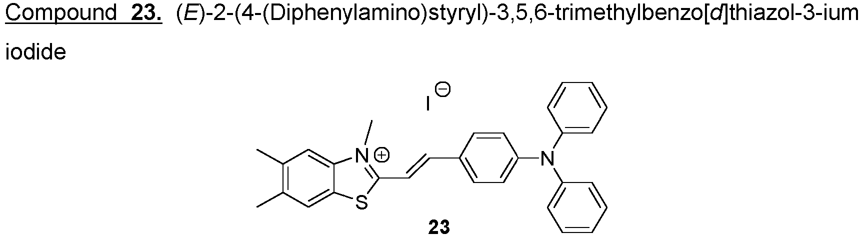

Pink solid (reaction time = 38 h, yield = 10%). ¹H NMR (400 MHz, DMSO-*d₆*): *δ* = 2.41 (3H, s, Ar-CH₃), 2.45 (3H, s, Ar-CH₃), 4.23 (3H, s, N⁺CH₃), 6.90 (2H, d, *J =* 8.8 Hz, H-3' and H-5'), 7.18 (4H, d, *J* = 7.2 Hz, (H-2"and H-6") x 2), 7.22 (2H, t, *J* = 7.2 Hz, H-4" x 2), 7.42 (4H, t, *J* = 7.2 Hz, (H-3" and H-5") x 2), 7.72 (1H, d, *J* = 16 Hz, Ha), 7.86 (2H, d, *J* = 8.8 Hz, H-2' and H-6'), 7.99 (1H, s, H-4), 8.04 (1H, d, *J* = 15.6 Hz, H-b), 8.09 (1H, s, H-7) ppm.

HRMS (ESI) *m*/*z:* [M-I⁻]⁺ calcd for C₃₀H₂₇N₂S⁺ 447.1889, found 447.1904.

### Example 3. Biological activity

Cell culture: Cell culture media were purchased from Gibco (Grand Island, NY, USA). Fetal bovine serum (FBS) was a product of Harlan-Seralab (Belton, U.K.). Supplements and other chemicals not listed in this section were obtained from Sigma Chemicals Co. (St. Louis, Mo., USA). Plastics for cell culture were supplied by Thermo Scientific^{™} BioLite. All tested compounds were dissolved in DMSO at a concentration of 10 mg/mL and stored at -20°C until use. Cell lines were maintained in Dulbecco's modified Eagle's medium (DMEM) containing glucose (1 g/L), glutamine (2 mM), penicillin (50 IU/mL), streptomycin (50 µg/mL) and amphotericin (1.25 µg/mL), supplemented with 10% FBS.

THP1 cells (human monocytic cell line) were maintained in RPMI-1640 medium supplemented with 10% heat-inactivated fetal bovine serum (hiFBS) and 5% penicillin/streptomycin (37 °C, 5% CO₂, and 100% humidity).

Parasite culture: *L. major* promastigotes (LucRE9) were cultured at 28 °C in 5% CO₂ in modified RPMI-1640 medium (Invitrogen, Carlsbad, CA) with 10% hiFBS.

Cytotoxicity assays: The cytotoxic effect of the compounds was evaluated in MRC-5 cell line, through alamarBlue^{®} assay (ThermoFisher scientific) (J. Med. Chem. 2018, 61, 1231-1240). The stock solutions of the compounds were prepared in DMSO, with the final DMSO concentration in each well maintained below 1%. Prior to compounds addition, 5 × 10³ cells per mL (MRC-5 cells) were seeded in 96-wells plates (100 µL/well) and incubated for 24h at 37 °C, 5% CO₂ to allow cell attachment. Subsequently, the compounds were added at various concentrations ranging from 0 to 100 µM, and the plates were incubated for 72 h. After the incubation period, 20 µL of resazurin solution (110 ng/ml) were added to each well and cells were incubated for 4 h. Then, cells were lysed with 50 µL/well of 3% SDS. The plate was incubated at 37 _{°}C for an additional hour and subsequently, the fluorescence intensity was measure at the Infinite F200 plate reader (TECAN Austria, GmbH), exciting at 550 nm and recording the emission at 590 nm. The obtained results were expressed as the concentration of compound that reduces cell growth by 50% compared to untreated control cells (IC50). Data are presented as the average of at least three independent measurements, each conducted in triplicate conditions.

Cytotoxicity assays on THP1: Cytotoxicity on THP1 was measured through the alamarBlue^{®} assay (Thermo Fisher Scientific). THP1 cells were seeded at a density of 3 × 10⁵ cells/mL in 96-well plates (100 µL/well) and treated with 20 ng/mL PMA (phorbol 12-myristate 13-acetate, Sigma) for 48 hours. Post-differentiation, the cells were washed and fresh medium was added (100 µL/well) before being reincubated. Following another 48 hours, the wells were washed again, and fresh medium with increasing concentrations of compounds was added (100 µL/well). Seventy-two hours later, 20 µL of resazurine solution (110 ng/mL) was added to each well, and the cells were incubated for an additional 4 hours at 37°C. Subsequently, 50 µL of 20% SDS was added to each well. The plate was incubated for an extra hour at 37°C before being analyzed for fluorescence using an Infinite F200 plate reader (TECAN Austria, GmbH). The excitation wavelength was set at 550 nm and the emission wavelength at 590 nm. Results are reported as the concentration of compounds that reduce cell growth by 50% compared to untreated control cells (IC₅₀), determined using SigmaPlot with a four-parameter logistic curve. Data represent the average of three independent experiments, each conducted in triplicate.

### In vitro antitrypanosomal activity against Trypanosoma brucei

Bloodstream forms (BSF) of *T. brucei brucei* 'single marker' S427 (S16) were grown at 37 °C, 5% CO₂ in HMI-9 medium supplemented with 10% (heat-inactivated fetal bovine serum, hiFBS). Drug susceptibility assay was performed as described in Carvalho L et al., 2015 (Antimicrob Agents Chemother. 2015 Oct;59(10):6151-60). Briefly, parasites (1 × 10⁴ BSF per mL) were incubated in 96-well plates with increasing concentration of drugs/compounds for 72 h at 37 °C, 5% CO₂ in culture medium. Cell proliferation was determined using the alamarBlue^{®} assay (B. Raz, et al., Acta Trop 68 (1997) 139-147). The Alamar Blue assay is used to determine drug sensitivity of African trypanosomes (*T. b. rhodesiense* and *T. b. gambiense*) *in vitro.* Fluorescence was recorded with an Infinite^{®} F200 microplate reader (Tecan Austria GmbH, Austria) equipped with 550 and 590 nm filters for excitation and emission wavelengths, respectively. The results are expressed as the concentration of compound that reduces cell growth by 50% compared to untreated control cells (IC₅₀). Data were presented as the average of at least three independent measurements, each conducted in triplicate conditions.

### In vitro antileishmanial activity against Leishmania major promastigotes

The anti-leishmanial activity of the compounds was determined using MTT-based assay (Sigma-Aldrich) (Antimicrob Agents Chemother 2011, 55, 3838-3844). The stock solutions of the compounds were prepared in DMSO with a final DMSO percentage in each well maintained below 1%. 4 × 10⁶ parasites per mL were incubated at 28 °C in 96-well plates (50 µL/well) either alone or in the presence of increasing concentration of compounds DMSO for 72 h. After the incubation period, 10 µL of MTT (5 mg/ml) was added to each well and parasites were incubated for 4 h at 28°C. Subsequently, cell lysis was performed using 50 µL/well of 20% SDS. The plate was then incubated at 37 °C for an additional hour and then absorbance was measured at a wavelength of 540 using an Infinite F200 plate reader (TECAN Austria, GmbH). The IC₅₀ was calculated as described above. Data were presented as the average of at least three independent measurements, each conducted in triplicate conditions.

### In vitro antileishmanial activity against Leishmania major amastigotes

The antiparasitic activity of the compounds against the intracellular form of *Leishmania major* was evaluated using the Luciferase Assay System kit from Promega. THP1 cells were seeded at a density of 3 × 10⁵ cells/mL in 96-well plates (100 µL/well) and treated with 20 ng/mL PMA (phorbol 12-myristate 13-acetate, Sigma) for 48 hours to induce differentiation. Post-differentiation, the cells were washed and fresh medium was added (100 µL/well) before being reincubated. Following an additional 24 hours, the wells were washed, and 50 µL of L. major-LucRE9 parasites at a density of 1.5 × 10⁶ parasites/mL (suspended in RPMI-1640 medium 5% hiFBS and 5% penicillin/streptomycin) were added to the wells. The plates were then incubated at 35°C in 5% CO₂. After 24 hours, the wells were washed three times with PBS, and fresh medium with increasing concentrations of compounds was added (100 µL/well). After another 72 hours of incubation, the cells were then treated with 25 µL of lysis buffer and stored at -80°C. Following 24 hours, 25 µL of luciferase enzyme substrate was added to each well, and the luciferase activity of the amastigotes was determined using a TECAN Infinite F200 microplate reader. Results are reported as the concentration of compounds that reduce parasite growth by 50% compared to untreated control infected macrophages (IC₅₀), determined using SigmaPlot with a four-parameter logistic curve. Data represent the average of three independent experiments, each conducted in triplicate.

Antitrypanosomal and antileishmanial activity of prepared compounds on *Trypanosoma brucei* and on promastigotes of *Leishmania major,* respectively.

**Table 3.**

| | IC₅₀ (uM) | | | Selectivity Index (SI) | |
|---|---|---|---|---|---|
| **Compd** | *T. brucei* | *L. major* | MRC-5 | *T.brucei* | *L.major* |
| **1** | **0.000019 ± 0.0000053** | 0.02 ± 0.013 | 1.52 ± 0.37 | **79206.5** | 62.7 |
| **2** | 0.34 ± 0.13 | 0.02 ± 0.011 | 5.13 ± 0.76 | 15.1 | 252.0 |
| **3** | **0.00026** ± **0.00017** | **0.0065** ± **0.0029** | 0.08 ± 0.03 | 294.5 | 12.0 |
| **4** | **0.00029 ± 0.00019** | **0.007** ± **0.002** | 14.01 ± 1.71 | **48310** | **2001.4** |
| **5** | **0.00054** ± **0.00029** | **0.012 ± 0.03** | 45.47 ± 8.42 | **84203** | **3789.2** |
| **6** | **0.00067 ± 0.00001** | **0.0082 ± 0.0012** | 15.12 ± 1.27 | **22567** | **1843.9** |
| **7** | **0.00041 ± 0.00001** | **0.0065 ± 0.0010** | 0.0044 ± 0.0002 | 10.73 | <1 |
| **8** | 0.036 ± 0.011 | **0.0083 ± 0.006** | 15.60 **±** 2.68 | 434 | **1879** |
| **9** | 0.0058 ± 0.001 | **0.0073 ± 0.004** | 14.65 ± 0.15 | **2547** | **2006** |
| **10** | 0.0066 ± 0.0017 | 0.034 ± 0.007 | 15.16 ± 0.58 | **2297** | 445 |
| **11** | 0.796 ± 0.133 | 0.162 ± 0.041 | 40.89 ± 3.52 | 51 | 252 |
| **12** | 0.989 ± 0.139 | 0.233 ± 0.109 | 57.44 ± 0.47 | 58 | 246 |
| **13** | **0.00092** ± **0.00004** | **0.010 ± 0.006** | 20.74 ± 0.93 | **22474** | **2074** |
| **14** | 0.191 ± 0.022 | 0.024 ± 0.003 | 21.93 t 0.19 | 115 | 913 |
| **15** | 0.129 ± 0.061 | **0.0078** ± **0.0023** | 18.07 ± 1.70 | 140 | **2316** |
| **16** | 0.459 ± 0.095 | 0.048 ± 0.028 | 24.79 **±** 2.74 | 54 | 516 |
| **17** | 0.078 ± 0.008 | 0.013 ± 0.008 | 5.98 ± 0.14 | 76 | 460 |
| **18** | 0.011 ± 0.004 | **0.0096** ± **0.0039** | 7.76 ± 0.83 | 684 | 808 |
| **19** | 0.0051 ± 0.0002 | 0.044 ± 0.007 | 2.88 ± 0.31 | 564.7 | 65.5 |
| **20** | 0.91 ± 0.18 | 0.08 ± 0.02 | 4.21 ± 0.49 | 4.63 | 52.63 |
| **21** | 12.40 ± 2.38 | 1.50 ± 0.03 | 12.87 ± 1.38 | 1.04 | 8.6 |
| **22** | 6.39 ± 0.25 | 2.77 ± 0.21 | 4.23 ± 0.11 | <1 | 1.53 |
| **23** | 0.077 ± 0.013 | 0.029 ± 0.01 | 0.53 ± 0.11 | 6.88 | 18.3 |

The selectivity index (SI) was calculated related to a healthy cell line (MRC-5) as IC₅₀ MRC-5 / IC₅₀ *T.brucei* or IC₅₀ MRC-5 / IC₅₀ *L.major.*

Antileishmanial activity of prepared compounds on amastigotes of *Leishmania major.*

**Table 4.**

| | | IC₅₀ (uM) | | Selectivity Index (SI) | |
|---|---|---|---|---|---|
| **Compd** | *L. major* | *THP-1* | MRC-5 | *THP-1* | *MRC-5* |
| **1** | **0.000485** ± **0.000617** | 22.4 ± 3.45 | 1.52 ± 0.37 | **46151** | **3132** |
| **2** | 0.0281 ± 0.00473 | 0.93 ± 0.02 | 5.13 ±0.76 | 33 | 182 |
| **3** | -- | -- | 0.08 ± 0.03 | -- | -- |
| **4** | **0.000374** ± **0.000457** | 21.39 ± 1.65 | 14.01 ± 1.71 | **57146** | **37429** |
| **5** | 0.0911 ± 0.1239 | 29.96 ± 1.06 | 45.47 ± 8.42 | 329 | 499 |
| **6** | 0.0040 ± 0.0016 | 21.18 ± 0.91 | 15.12 ± 1.27 | 5267 | 3760 |
| **7** | -- | -- | 0.0044 ± 0.0002 | -- | -- |
| **8** | **0.00198 ± 0.00103** | 21.87 ± 0.3 | 15.60 ± 2.68 | **11101** | **7894** |
| **9** | **0.00130 ± 0.00178** | 6.7 ± 0.49 | 14.65 ± 0.15 | **5153** | **11261** |
| **10** | **0.00120 ± 0.00029** | 14.01 ± 6.17 | 15.16 t 0.58 | **11620** | **12574** |
| **11** | **0.000463** ± **0.000363** | 67.18 ± 1.61 | 40.89 ± 3.52 | **145097** | **88315** |
| **12** | 0.0339 ± 0.0040 | 68.02 ± 1.48 | 57.44 ± 0.47 | 2001 | 1689 |
| **13** | 0.0043 ± 0.0004 | 20.72 ± 2.38 | 20.74 ± 0.93 | 4829 | 4834 |
| **14** | **0.0001216 ± 0.0000447** | 25.21 ± 4.22 | 21.93 ± 0.19 | **207319** | **180345** |
| **15** | **0.0000194 ± 0.0000171** | 19.99 ± 0.57 | 18.07 ± 1.70 | **1030412** | **931443** |
| **16** | 0.002437 ± 0.003798 | 52.14 ± 7.22 | 24.79 ± 2.74 | 21395 | 10172 |
| **17** | 0.01007 ± 0.0172 | 20.55 ± 0.07 | 5.98 ± 0.14 | 2040 | 594 |
| **18** | **0.0000343 ± 0.0000067** | 4.18 ± 0.82 | 7.76 ± 0.83 | **121866** | **226239** |
| **19** | **0.000664 ± 0.0001426** | **6.03 ± 0.45** | **2.88 ± 0.31** | **9075** | **4334** |
| **20** | **0.00392 ± 0.00548** | **0.89 ± 0.13** | **4.21 ± 0.49** | **227** | **1073** |
| **21** | -- | -- | 12.87 ± 1.38 | -- | -- |
| **22** | -- | -- | 4.23 ± 0.11 | -- | -- |
| **23** | -- | -- | 0.53 ± 0.11 | -- | -- |

The selectivity index (SI) was calculated related to a healthy cell line (MRC-5) as IC₅₀ THP-1 / IC₅₀ *L. major* or IC₅₀ MRC-5 / IC₅₀ *L.major.*

## Claims

1. Compound of formula (I), or a pharmaceutically acceptable salt thereof: 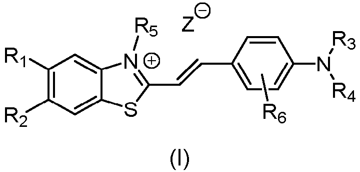 wherein
R₁ and R₂ are each independently selected from H, halogen, -O-(C₁-C₄)-alkyl or C₁-C₄ alkyl,
R₃ and R₄ are each independently selected from:
optionally substituted C₁-C₈ alkyl and phenyl,
R₃ and R₄ form a five- or six-membered ring with the N to which they are bonded and with the phenyl ring that is bonded to that N, and
R₃ and R₄ form, together with the N to which they are bonded, a five- or six-membered aliphatic or aromatic ring,
R₅ is an optionally substituted C₁₋₈ alkyl,
R₆ is a H, halogen or an optionally substituted C₁₋₈ alkyl,
Z is a counterion to offset the positive charge of the nitrogen of the aromatic ring being zero the final charge of the compound of formula (I),
for use in the treatment and/or prevention of parasitic infections.

2. Compound of formula (I) for use, according to claim 1, wherein R₁ and R₂ are each independently H or halogen.

3. Compound of formula (I) for use, according to claim 2, wherein the halogen is chlorine or fluorine.

4. Compound of formula (I) for use, according to claim 1 or 2, wherein R₃ and R₄ are each independently C₁-C₈ alkyl.

5. Compound of formula (I) for use, according to claim 4, wherein R₃ and R₄ are each independently C₁-C₄ alkyl, preferably ethyl or methyl

6. Compound of formula (I) for use, according to any of previous claims, wherein R₅ is independently C₁-C₈ alkyl, preferably C₁-C₄ alkyl, even more preferably propyl, ethyl or methyl.

7. Compound of formula (I) for use, according to any of previous claims, wherein R₆ is independently H, halogen or C₁-C₈ alkyl, preferably, R₆ is H or F.

8. Compound of formula (I) for use, according to any of previous claims, wherein R₆ is in position ortho with respect to the -NR₃R₄ group.

9. Compound of formula (I) for use, according to any of previous claims, Z⁻ is selected from: halide, acetate, besylate, benzoate, carbonate, bicarbonate, benzenesulphonate, bitartrate, citrate, estearate, phosphate, fumarate, glucuronate, glycolate, hexanoate, hydroxynaphtoate, lactate, mesylate, nitrate, nitrite, octanoate, oleate, pamoate, pantotenate, propionate, polygalacturonate, salicylate, succinate, sulphate, tartrate, teoclate, tosylate, triflate, tetraphenylborate and trifluoroacetate.

10. Compound of formula (I) for use, according to any of previous claims, wherein the compound is selected from the group:

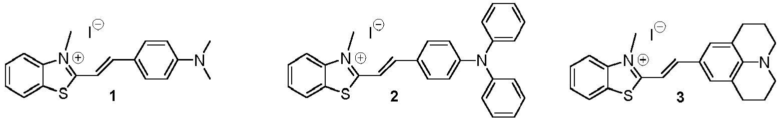

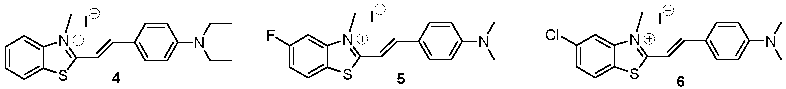

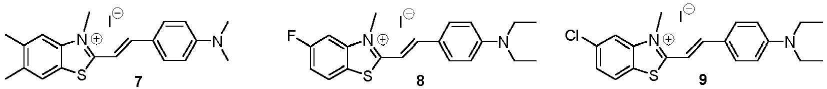

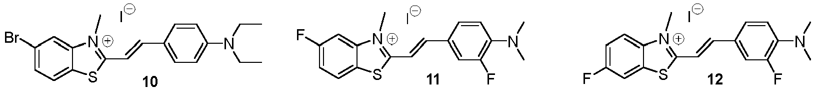

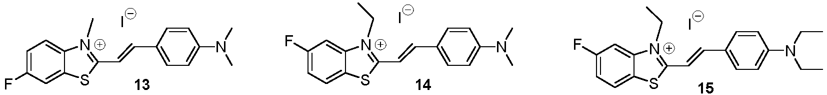

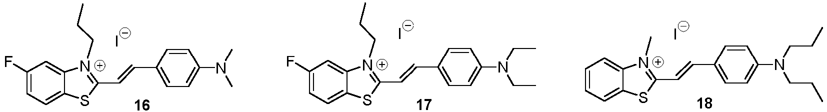

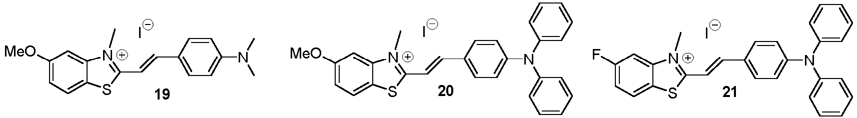

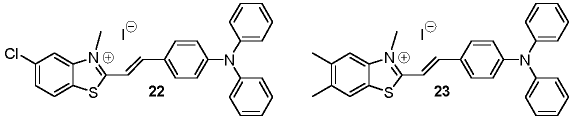

11. Compound of formula (I) for use, according to any of previous claims, wherein the parasitic infection is selected from: african trypanosomiasis, chagas, leishmaniasis, blastocystosis, cryptosporidiosis, amoebiasis, giardiasis, sarcosystosis, toxoplasmosis, trichomoniasis and malaria.

12. Compound of formula (I) for use, according to any of previous claims, wherein the the parasitic infection is Leishmaniasis caused by *Leishmania major, Leishmania donovani* or *Leishmania infantum* parasites.

13. Compound of formula (I) selected from:

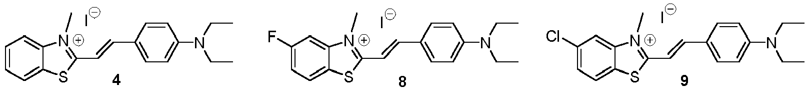

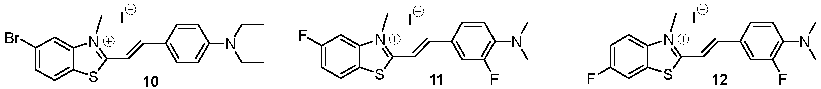

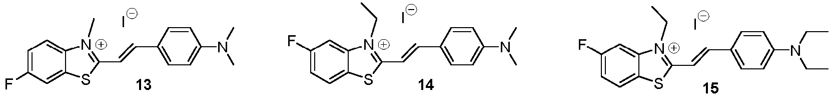

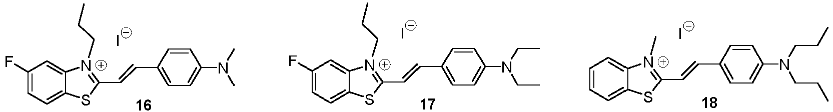

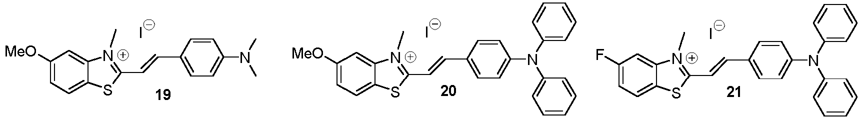

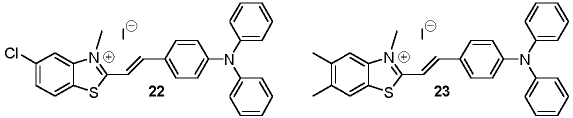

14. Compound of formula (I), according to claim 13, for use as a medicament.

15. Pharmaceutical composition comprising at least one compound of formula (I) as defined in claim 13, or a pharmaceutically acceptable salt thereof, together with a pharmaceutically acceptable excipients, adjuvants and/or carriers.
